# EUROPEAN PATENT SPECIFICATION

(11) **EP 1 938 760 B1**
(45) Date of publication and mention of the grant of the patent: **12.11.2014**
(21) Application number: 06810746.5
(22) Date of filing: 28.09.2006
(51) Int. Cl.: A61B 17/04

(54) **SUTURING DEVICE**
NAHTVORRICHTUNG
DISPOSITIF DE SUTURE

(30) Priority: 28.09.2005 US 238016; 28.09.2005 US 238017
(43) Date of publication of application: 02.07.2008
(73) Proprietor: OLYMPUS MEDICAL SYSTEMS CORP., Tokyo 151-0072 (JP)
(72) Inventor: MIKKAICHI, Takayasu, Fuchu-shi Tokyo 1830033 (JP); SUZUKI, Takayuki, Yokohama-shi, Kanagawa 2260027 (JP); KAJI, Kunihide, Hachioji-shi, Tokyo 1920023 (JP); SHIONO, Junji, Yokohama-shi, Kanagawa 2260011 (JP); HAYASHI, Kensuke, Yokohama-shi, Kanagawa 2360053 (JP); SATO, Masatoshi, Yokohama-shi, Kanagawa 2260011 (JP); IWASAKA, Masayuki, Tama-shi, Tokyo 2060034 (JP)
(74) Representative: von Hellfeld, Axel
(86) International application number: PCT/JP2006/319299
(87) International publication number: WO 2007/037326

(56) References cited:
- EP-A1- 1 484 021
- WO-A1-01/70328
- WO-A2-2005/065412
- JP-A- 2000 516 513
- JP-A- 2002 233 530
- JP-A- 2004 358 045
- US-A- 4 235 238
- US-A- 5 948 002
- US-A1- 2005 059 984

## Description

### [TECHNICAL FIELD]

The present invention relates to a suture instrument that uses an endoscope, for example, relating to a suture instrument that sutures a perforation that is formed in the wall of a hollow organ.

Priority is claimed on U.S. Patent Application No. 11/238016 and 11/238017 filed on September 28, 2005.

### [BACKGROUND ART]

In the case of performing treatment in a body of a patient, the treatment can be performed by incising the body of the patient by surgical operation, or by oral endoscopic treatment or transanal endoscopic treatment. A method for suturing a perforation formed in an abdominal area by surgical operation is disclosed in FIGS. 6a to 6c of - Patent Document 1. According to this suturing method, a needle is thrust into the tissue around the perforation, and an anchor equipped with a suture thread is then extruded from the needle. After the needle is drawn out from the tissue, two suture threads across the perforation are knotted together to close the perforation.

The treatment using an endoscope is carried out by passing a forceps, high-frequency treatment instrument, incision instrument, suture tool or the like through a channel of the endoscope. When the medical treatment is carried out by using an endoscope inserted into a hollow organ through a natural opening of a living body such as the mouth, anus, or the like, for example, a hole is formed by removing the tissue from the abdominal cavity or incising the tissue in the abdominal cavity, and the medical treatment is then carried out by approaching the abdominal cavity through this hole from the inside of the hollow organ. After performing the medical treatment, the formed hole is sutured by a suture tool.

A method for suturing in a hollow organ is disclosed in FIGS. 6 to 9 of Patent Document 2, for example. According to this suturing method, the tissue is drawn into an overtube, and a needle is then thrust through this tissue from the proximal side to the distal end side thereof. From the inside of the needle, an anchor equipped with a suture thread is pushed out to the distal end side of the tissue. After that, when the needle is pulled out, the suture thread penetrates through the tissue, and so the tissue is tightened up by this suture thread. There is also a method disclosed in FIG 1, FIG 4, FIGS. 5A to 5C of Patent Document 3. According to this method, a flexible endoscope is inserted into the vicinity of a perforation via the mouth or the anus. The tissue around the perforation is aspirated by a tube of the flexible endoscope. When an O-ring provided at the outside of the tube is pushed out from the distal end of the tube, the aspirated tissue is clamped by the O-ring.

Here, it is preferable to be able to control the deployment of each anchor. For example, in a hard endoscope treatment tool, it is known to provide in the control portion on the hand side a plurality of notches that restrict the stroke of a pusher that pushes out anchors to a fixed amount. (For example, refer to Patent Document 4.)

A suturing instrument according to the preamble of claim 1 is provided in EP 1484021 A1.
Patent Document 1: U.S. Patent No. 6,066,146
Patent Document 2: Japanese Unexamined Patent Application, First Publication No. 2004-601
Patent Document 3: U.S. Patent No. 5,297,536
Patent Document 4: U.S. Patent No. 5,507,754

### [DISCLOSURE OF THE INVENTION]

### [PROBLEM TO BE SOLVED BY THE INVENTION]

However, in a flexible endoscope treatment tool, even if notches are provided in the control portion on the hand side, since a long flexible portion exists between the control portion and the anchor, it is not possible to press the anchor by the same amount even when advancing the pusher by one notch.

The present invention was achieved in view of the above circumstances, and has as its main object to provide a suture instrument that can reliably push out anchors one at a time.

### [DISCLOSURE OF THE INVENTION]

A suture instrument according to claim 1 is provided. A first aspect is a suture instrument that is inserted into a body and ejects an elongated anchor to be engaged to tissue, including: an elongated member that extends from a proximal end on a hand side to a remote end that is introduced to tissue and has flexibility; a needle that is provided at the remote end of the elongated member, has at the distal end a sharp tip that is capable of puncturing tissue, is capable of housing a plurality of the anchors inside, and is provided with a side hole through which the anchors are capable of passing; a pusher whose remote end abuts the anchor that is housed in the needle and is disposed in a manner to be capable of moving in the axial direction of the elongated member; and a push-out control portion that is provided in the needle or the pusher and changes the movement direction of the anchor that has been moved to a formation position of the side hole by the pusher from the length-wise direction of the needle to a direction heading toward the side hole.

In this suture instrument in accordance with the first aspect, a plurality of anchors are moved in the lengthwise direction of the needle toward the distal end thereof by moving the pusher forward. The push-out control portion first causes the first anchor that has been moved to the formation position of the side hole to move toward the side hole, and causes this anchor to be pushed out from the side hole. The second anchor is moved to the formation position after the first anchor has been pushed out, and then pushed out from the side hole by the push-out control portion.

A second aspect is the suture instrument according to the first aspect, wherein the push-out control portion is a plate spring that is provided in the needle and projects toward the side hole.

In this suture instrument in accordance with the second aspect, the anchor that has been moved to the formation position of the side hole is pushed out from the side hole by being biased by the plate spring.

A third aspect is the suture instrument according to a first aspect, wherein the push-out control portion is a erecting hook that is attached to the needle in a manner to freely rotate and is capable of rising toward the side hole.

In this suture instrument in accordance with the third aspect, when the anchor has moved to the formation position of the side hole, the anchor is pushed out from the side hole by raising the erecting hook.

A fourth aspect is the suture instrument according to a first aspect, wherein the push-out control portion is a slanted face that is obliquely provided at the distal end of the pusher in a manner facing the side hole and abuts the base end portion of the anchor that is housed at the base end side among the two anchors, and at the distal end portion of the anchor that is housed at the base end side is formed a face that slants toward the side hole and abuts the base end portion of the anchor that is housed at the distal end side.

In this suture instrument in accordance with the fourth aspect, since the contact face of the pusher and the base end side anchor obliquely face the side hole, when the base end side anchor reaches the formation position of the side hole, the force of pushing the anchor is obliquely applied toward the side hole. As a result, the base-end side anchor is pushed out from the side hole. Since the abutting faces of the distal end side anchor and the base-end side anchor slant toward the side hole, the force of the pusher is obliquely transmitted to the distal end side anchor to be pushed out from the side hole.

A fifth aspect is the suture instrument according to the first aspect, wherein the needle additionally has an opening portion at the sharp end portion, the push-out control portion consists of a projection that protrudes so as to press the distal end side first anchor toward the side hole, the distal end side first anchor has an outer shape to be pushed out from the side hole by being guided by the projection, and the base-end side second anchor has an outer shape that enables it to be pushed out from the opening portion without interference from the projection.

In this suture instrument in accordance with the fifth aspect , when the pusher is moved forward, the first anchor is moved toward the side hole by the projection and pushed out from the side hole. In contrast, the second anchor is pushed out from the distal end side opening portion without interference from the protrusion.

A sixth aspect is a suture instrument that is inserted into a body and ejects an elongated anchor to be engaged to tissue, including: an elongated member that extends from a proximal end on a hand side to a remote end that is introduced to tissue and has flexibility; a needle that is provided at the remote end of the elongated member, has at the distal end a sharp tip that is capable of puncturing tissue, and is capable of housing a plurality of the anchors inside; and a side hole that is provided in the side portion of the needle along the lengthwise direction of the needle; wherein by compressing the anchors in a direction that intersects at right angles the lengthwise direction thereof, the anchors are housed in the needle in the state of the dimension in this direction decreasing and the dimension in the lengthwise direction being extended.

In this suture instrument in accordance with the sixth aspect, the anchors are housed in the needle in the state of being deformed. When the anchors are moved to the formation position of the side hole by moving the pusher forward, the anchors revert to their original shape are released from the side hole.

A seventh aspect is a suture instrument that is inserted into a body and ejects an elongated anchor to be engaged to tissue, including: an elongated member that extends from a proximal end on a hand side to a remote end that is introduced to tissue and has flexibility; a needle that is provided at the remote end of the elongated member, has at the distal end a sharp tip that is capable of puncturing tissue, and is capable of housing a plurality of the anchors inside; and a side hole that is provided in the side portion of the needle along the lengthwise direction of the needle and has an opening length that is shorter than the extended length of one anchor.

In this suture instrument in accordance with the seventh aspect, the anchors are curved by moving the pusher forward. When the length of the anchor along the lengthwise direction of the needle becomes shorter than the side hole, the anchor is pushed out from the side hole.

An eighth aspect is a suture instrument that is inserted into a body and ejects an elongated anchor to be engaged to tissue, including: an elongated member that extends from a proximal end on a hand side to a remote end that is introduced to tissue and has flexibility; a needle that is provided at the remote end of the elongated member, has at the distal end a sharp tip that is capable of puncturing tissue, and is capable of housing a first anchor and a second anchor inside; a first pusher that pushes out the first anchor that is housed at the distal end side from the needle; and a second pusher that is provided to freely move forward or backward with respect to the first pusher and pushes out the second anchor from the needle.

In this suture instrument in accordance with the eighth aspect, by operating the first pusher and the second pusher in turn, the two anchors are pushed out from the needle in turn.

A ninth aspect of the present invention is the suture instrument according to the eighth aspect, wherein a housing portion that disposes the second anchor is provided in the first pusher, and the second pusher is passed in a manner to freely move forward or backward in the first pusher.

In this suture instrument in accordance with the ninth aspect, the first pusher pushes out the first anchor while housing the second pusher. Thereafter, when the second pusher is moved forward with respect to the first pusher, the second pusher is pushed out from the first pusher and the needle.

A tenth aspect is the suture instrument according to the eighth aspect, wherein the first pusher is passed in the second pusher and, by passing through a through hole that is formed in the second anchor, pushes out the first anchor.

In this suture instrument in accordance with the tenth aspect, when the first pusher is moved forward with respect to the second pusher, the first pusher, by passing through the second anchor, pushes out the first anchor from the needle. The second pusher can push out the second anchor by moving forward the second anchor.

An eleventh aspect is the suture instrument according to the tenth aspect, wherein the second pusher is passed in the lengthwise direction of the needle in a manner to be capable of being pushed and pulled therethrough, and the base end portion of the first pusher is engaged with the second pusher in a manner to be capable of screwing thereon.

In this suture instrument in accordance with the eleventh aspect, by screwing the first pusher onto the second pusher, the first anchor is pushed out. The second anchor is pushed out from the needle by pushing in the second pusher. By differentiating the operation methods when pushing out the two pushers, the anchors can be reliably pushed out one at a time.

A twelfth aspect is a suture instrument that is inserted into a body and ejects an elongated anchor to be engaged to tissue, including: an elongated member that extends from a proximal end on a hand side to a remote end that is introduced to tissue and has flexibility; a needle that is provided at the remote end of the elongated member, has at the distal end a sharp tip that is capable of puncturing tissue, is capable of housing a plurality of anchors inside, and has provided at the sharp tip an opening portion that is capable of pushing out the anchors; a pusher that pushes out the anchors from the needle through the opening portion; and a side hole that is provided in an extended manner from the opening portion of the needle and through which a suture thread that is attached to each of the anchors is passed, being formed in a shape that enables the anchors to be pushed out from the opening portion of the needle one at a time.

This suture instrument in accordance with the twelfth aspect has a side hole as a push-out control portion for pushing out the anchors one at a time. The pushing out of the anchors is controlled by the shape of the side hole.

A thirteenth aspect is the suture instrument according to the twelfth aspect, wherein a portion of the side hole that passes the suture thread extending from a first anchor that is housed at the distal end side has a crooked shape so as to shift in the circumferential direction of the needle with respect to a portion that passes the suture thread extending from a second anchor that is housed at the base-end side, and the pusher has a distal end portion that engages in the circumferential direction with the second anchor.

Since the side hole of this suture instrument in accordance with the thirteenth aspect of the present invention has a crooked shape, it is necessary to rotate the second anchor in the circumferential direction to push it out. For this reason, a plurality of anchors being simultaneously pushed out does not occur.

A fourteenth aspect is the suture instrument according to the twelfth aspect, wherein a portion of the side hole that passes the suture thread extending from a first anchor that is housed at the distal end side has a curved shape so as to shift in the circumferential direction of the needle with respect to a portion that passes the suture thread extending from a second anchor that is housed at the base-end side.

Since this suture instrument in accordance with the fourteenth aspect pushes out the second anchor along the curved shape of the side hole, the force required to push out the second anchor is greater than the force required to push out the first anchor. For this reason, a plurality of anchors being simultaneously pushed out does not occur.

A fifteenth aspect is the suture instrument according to the twelfth aspect, wherein a narrowed portion in which the opening width is partially narrowed is formed in the side hole.

Since the narrowed portion in this suture instrument in accordance with the fifteenth aspect of the present invention serves as resistance, a plurality of anchors being simultaneously pushed out does not occur.

A sixteenth aspect is the suture instrument according to any one of the twelfth aspect to the fifteenth aspect, wherein the anchors have a projection that is inserted into the side hole.

In this suture instrument in accordance with the sixteenth aspect, by guiding the anchor projection to the distal end side in accordance with the shape of the side hole, the simultaneous pushing out of the plurality of anchors is prevented.

A seventeenth aspect is a suture instrument that is inserted into a body and ejects an elongated anchor to be engaged to tissue, including: an elongated member that extends from a proximal end on a hand side to a remote end that is introduced to tissue and has flexibility; a needle that is provided at the remote end of the elongated member, has at the distal end a sharp tip that is capable of puncturing tissue, is capable of housing a plurality of anchors inside, and has provided at the sharp tip an opening portion that is capable of pushing out the anchors; and a pusher that pushes out the anchors from the needle through the opening portion; wherein the needle is manufactured to have resilience with a projection that reduces the inner diameter being formed therein, and the first anchor that is disposed at the distal end side has an outer diameter that is capable of passing the projection while the second anchor that is disposed at the base-end side has an outer diameter that interferes with the projection.

In this suture instrument in accordance with the seventeenth aspect, when the pusher is moved forward, the first anchor passes the projection and is smoothly pushed out, but the second anchor stops due to interference with the projection. Since it is necessary to apply an even greater force to push out the second anchor, a plurality of anchors being simultaneously pushed out does not occur.

An eighteenth aspect is a suture instrument that is inserted into a body and ejects an elongated anchor to be engaged to tissue, including: an elongated member that extends from a proximal end on a hand side to a remote end that is introduced to tissue and has flexibility; a needle that is provided at the remote end of the elongated member, has at the distal end a sharp tip that is capable of puncturing tissue, is capable of housing a plurality of anchors inside, and has provided at the sharp tip an opening portion that is capable of pushing out the anchors; a pusher that pushes out the anchors from the needle through the opening portion; a first connecting portion that connects a first anchor that is disposed at the distal end side and a second anchor that is disposed at the base-end side and is breakable; and a second connecting portion that connects the second anchor and the pusher and is breakable.

In this suture instrument in accordance with the eighteenth aspect, the first and second anchors are connected to the pusher. When placing the first anchor, the first connecting portion is broken. When placing the second anchor, the second connecting portion is broken after the first anchor has been placed.

A nineteenth aspect is a suture instrument that is inserted into a body and ejects an elongated anchor to be engaged to tissue, including: an elongated member that extends from a proximal end on a hand side to a remote end that is introduced to tissue and has flexibility; a needle that is provided at the remote end of the elongated member, has at the distal end a sharp tip that is capable of puncturing tissue, is capable of housing a plurality of anchors inside, and has obliquely provided at the sharp tip an opening portion that is capable of pushing out the anchors; and a pusher that pushes out the anchors from the needle through the opening portion; wherein the needle is capable of housing two anchors arranged in the radial direction along the slanting direction of the sharp tip.

In this suture instrument in accordance with the nineteenth aspect, since the anchor at the base-end side of the slanting opening portion is protruded from the needle first, it is possible to place this anchor first. The anchor at the distal end side of the slanting opening portion is protruded from the needle with a delay in accordance with the slant of the opening portion. For this reason, the two anchors being simultaneously pushed out from the needle does not occur.

A twentieth aspect is the suture instrument according to the nineteenth aspect, wherein the distal end portion of the pusher is capable of pushing each of the two anchors, and the base-end side of the sharp tip that slants protrudes.

In this suture instrument in accordance with the twentieth aspect, since the pusher protrudes the base-end side of the sharp tip that slants, it is possible to push out the anchor that is disposed at this position earlier than the other anchor.

A twenty-first aspect is a suture instrument that is inserted into a body and ejects an elongated anchor to be engaged to tissue, including: an elongated member that extends from a proximal end on a hand side to a remote end that is introduced to tissue and has flexibility; a needle that is provided at the remote end of the elongated member, has at the distal end a sharp tip that is capable of puncturing tissue, is capable of housing a plurality of anchors inside, and has obliquely provided at the sharp tip an opening portion that is capable of pushing out the anchors; and a pusher that pushes out the anchors from the needle through the opening portion; wherein the distal end portion of the pusher has recessed portions that are capable of housing each of the two anchors in the lengthwise direction of the needle, with the distal end side recessed portion being formed facing the opening direction of the opening portion, and the base-end side recessed portion formed facing the opposite side of the distal end side recessed portion.

In this suture instrument in accordance with the twenty-first aspect, the anchor that is disposed in the distal end side recessed portion is pushed out from the needle first. At this time, since the base-end side anchor is sandwiched between the base-end side recessed portion of the pusher and the needle, it is not pushed out from the needle.

A twenty-second aspect is a suture instrument that is inserted into a body and ejects an elongated anchor to be engaged to tissue, including: an elongated member that extends from a proximal end on a hand side to a remote end that is introduced to tissue and has flexibility; a needle that is provided at the remote end of the elongated member, has at the distal end a sharp tip that is capable of puncturing tissue, is capable of housing a plurality of anchors inside, and has provided at the sharp tip an opening portion that is capable of pushing out the anchors; and a pusher that pushes out the anchors from the needle; wherein a push-out control portion that is capable of engaging and disengaging with at least one of the anchors to cause the anchors to be pushed out one at a time from the opening portion is provided in the needle or the pusher.

In this suture instrument in accordance with the twenty-second aspect, the plurality of anchors is not pushed out simultaneously by engaging and disengaging the anchors and the push-out control portion.

A twenty-third aspect is the suture instrument according to the twenty-second aspect, wherein the push-out control portion consists of projections that are formed facing the inner side of the needle, and recessed portions that are capable of engaging and disengaging with the projections are provided on the anchors.

In this suture instrument in accordance with the twenty-third aspect, when pushing out the anchor that has the recessed portion, the recessed portion and the projections are engaged and disengaged by pushing the pusher.

A twenty-fourth aspect is the suture instrument according to the twenty-second aspect, wherein the push-out control portion is mounted on the needle and has a projection that passes through the needle to protrude toward the inner side thereof, and a recessed portions that is capable of engaging and disengaging with the projection is provided on the anchors.

In this suture instrument in accordance with the twenty-foourth aspect, when the push-out control portion is mounted on the needle, the projection of the push-out control portion is disposed to be capable of engaging and disengaging with the recessed portion of the anchors.

A twenty-fifth aspect is the suture instrument according to the twenty-second aspect, wherein the push-out control portion consists of an elongated spring that has a projection that is biased to be capable of engaging and disengaging with a recessed portion that is formed on the anchors.

In this suture instrument in accordance with the twenty-fifth aspect, to push out the anchors that have the recessed portion, the pusher is pushed in to cause the recessed portion and the projection to engage and disengage by deforming the push-out control portion.

A twenty-sixth aspect is the suture instrument according to any one of the twenty-third aspect to the twenty-fifth aspect, wherein two of the anchors are housed in the needle, and the recessed portion is formed in only one of the anchors.

In this suture instrument in accordance with the twenty-sixth aspect, since there is an anchor that engages with the push-out control portion and an anchor that does not engage with the push-out control portion, there is a difference in the operational feeling when pushing out these anchors, and so it is easy to push out the anchors one at a time.

A twenty-seventh aspect is the suture instrument according to the twenty-second aspect, wherein the push-out control portion is a spring that expands diameter in the radial direction when compressed in the lengthwise direction of the needle to release the engagement with the anchor.

In this suture device in accordance with the twenty-seventh aspect, the spring serves as a stopper to prevent protrusion of the anchors. To push out an anchor with which the spring is engaged, the spring is compressed. Thereby, the diameter of the spring widens, and the engagement between the spring and the anchor is released, so that if the anchor is pushed it will protrude from the needle.

A twenty-eighth aspect is the suture instrument according to the twenty-second aspect, wherein an intermediate member that freely engages and disengages with the second anchor is provided at the distal end portion of the pusher as a push-out control portion, and a side hole is provided that biases the intermediate member so that the second anchor engages with the intermediate member at the base end side of the needle and releases the bias of the intermediate member so that the engagement of the intermediate member and the second anchor is released at the distal end side of the needle.

In this suture instrument in accordance with the twenty-eighth aspect, to push out the first anchor, when the first anchor is pushed out, the second anchor moves forward together with the intermediate member, but since the intermediate member and the second anchor are engaged, the two anchors are not pushed out simultaneously.

A twenty-ninth aspect is the suture instrument according to the twenty-second aspect, wherein an intermediate member that freely engages and disengages with the second anchor is provided at the distal end portion of the pusher as a push-out control portion, and either one of the second anchor and the intermediate member is manufactured from a permanent magnet and the other is manufactured from a magnetic body

In this suture instrument in accordance with the twenty-ninth aspect, in order to push out the second anchor, since it is necessary to apply a force that overcomes the attraction between the permanent magnet and the magnetic body, it is possible to prevent the two anchors from being pushed out simultaneously.

A thirtieth aspect is the suture instrument according to the twenty-second aspect, wherein a hook is provided at the distal end portion of the pusher as a push-out control portion, is inserted and engaged with a recessed portion of an anchor that is housed in the base-end side, and when pulled out from the recessed portion, expands to be capable of pressing the base-end portion of the anchor.

In this suture instrument in accordance with the thirtieth aspect, the distal end side anchor is pushed out in the state of the hook being inserted into the recessed portion of the base-end side of the pusher. The base-end side anchor is pushed out after releasing the engagement between the hook and the recessed portion.

### [EFFECT OF THE INVENTION]

By providing the placement and structure according to the present invention in the distal end side of the needle or the pusher, when operating the pusher at the hand side, it becomes possible to reliably push out a plurality of anchors that are housed in the needle in a predetermined order one at a time. Since a delicate operation at the hand side becomes unnecessary, the procedure is simplified and the procedure efficiency improves.

### [BRIEF DESCRIPTION OF DRAWINGS]

FIG 1 is a drawing that shows the schematic configuration of an endoscope and a suture instrument.
FIG 2 is a cross-sectional view of the suture instrument and the distal end portion of the endoscope.
FIG 3 is a perspective view of the suture instrument and the distal end portion of the endoscope.
FIG 4 is a drawing that shows the constitution of a suture tool.
FIG. 5 is a cross-sectional view along the line A-A in FIG. 2.
FIG. 6 is a drawing that shows the step of inserting the endoscope into the stomach of a patient and observing an intended incision line from within the stomach.
FIG 7 is a drawing that shows the step of performing a treatment in an abdominal cavity through a perforation.
FIG 8 is a drawing that shows the position of thrusting the needle.
FIG. 9 is a drawing that describes the operation of pushing out the first anchor.
FIG 10 is a drawing that shows the first anchor being pushed out after the needle has penetrated.
FIG. 11 is a drawing that shows the mounting of the suture tool.
FIG. 12 is a drawing that shows forceps tightening the suture tool.
FIG. 13 is a drawing that shows the step of tightening the suture tool with the outer sheath of the forceps.
FIG. 14 is a drawing that shows the mounting of the suture tool.
FIG. 15 is a drawing that shows the tightened suture tool.
FIG. 16 is a drawing that shows the tightened suture tool.
FIG 17 is a drawing that shows the step of thrusting the needle obliquely at the inner circumferential face of the perforation.
FIG. 18 is a drawing that shows the anchor being pushed out after the needle has penetrated.
FIG. 19 is a drawing that shows the mounting of the suture tool.
FIG 20 is a drawing that shows the membrane on the inner portion side of the stomach being resected in the area of the perforation.
FIG 21 is a drawing that shows the step of thrusting the needle into the face that is exposed by resecting the membrane.
FIG 22 is a drawing that shows the mounting of the suture tool.
FIG 23 is a drawing that shows the tightened suture tool.
FIG 24 is a drawing that shows the step of resecting the membrane and thrusting the needle into the surface that is made to swell by a local injection in that area.
FIG. 25 is a drawing that shows the mounting of the suture tool.
FIG 26 is a drawing that shows the tightened suture tool.
FIG 27 is a drawing that shows the membrane of the stomach resected.
FIG 28 is a perspective view of the suture instrument, grasping forceps and the distal end portion of the endoscope.
FIG 29 is a drawing that shows the step of elevating the area where the membrane is resected with the grasping forceps.
FIG 30 is a drawing that shows the step of inserting the needle into the elevated membrane.
FIG 31 is a drawing that shows the tightened suture tool.
FIG 32 is a drawing that shows varieties of examples of elevating the membrane and placing the anchors.
FIG 33 is a drawing that shows varieties of examples of elevating the membrane and placing the anchors.
FIG 34 is a drawing that shows varieties of examples of elevating the membrane and placing the anchors.
FIG 35 is a cross-sectional view of the distal end portion of the suture instrument that houses curved anchors.
FIG. 36 is a drawing that describes the operation of pushing out the first anchor from the needle.
FIG 37 is a cross-sectional view of the distal end portion of the suture instrument that has a plate spring in the needle.
FIG. 38 is a drawing that describes the operation of pushing out the first anchor from the needle.
FIG 39 is a cross-sectional view of the distal end portion of the suture instrument that has an erecting hook in the needle.
FIG 40 is a cross-sectional view of the distal end portion of the suture instrument that is provided with a needle in which the routes for pushing out two anchors differ.
FIG 41 is a cross-sectional view along the line B-B in FIG 40.
FIG 42 is a drawing that describes the operation of pushing out the first anchor.
FIG 43 is a drawing that describes the operation of pushing out the second anchor.
FIG 44 is a cross-sectional view of the distal end portion of the suture instrument in the case of the anchors being permanent magnets.
FIG 45 is a drawing that describes the operation of pushing out the first anchor.
FIG 46 is a cross-sectional view of the distal end portion of the suture instrument that has pushers corresponding to two anchors of different diameters.
FIG 47 is a drawing that describes the operation of pushing out the first anchor.
FIG 48 is a drawing that describes the operation of pushing out the second anchor.
FIG 49 is a perspective view of the distal end portion of the suture instrument that houses two anchors in parallel.
FIG 50 is a perspective view of the distal end portion of another example of the suture instrument that houses two anchors in parallel.
FIG. 51 is a cross-sectional view that shows the constitution of the suture instrument that is provided with a pusher that passes through the second anchor to be capable of pushing out the first anchor.
FIG 52 is a drawing that describes the operation of pushing out the first anchor.
FIG 53 is a drawing that describes the operation of pushing out the second anchor.
FIG 54 is a cross-sectional view of the distal end portion of the suture instrument in which the two anchors are connected to the pusher.
FIG 55 is a cross-sectional view along the line C-C in FIG 54.
FIG 56 is a drawing that describes the operation of pushing out the first anchor and causing it to separate from the second anchor.
FIG 57 is a drawing that describes the operation of pushing out the second anchor and causing it to separate from the pusher.
FIG 58 is a drawing that shows the distal end portion of the suture instrument that has a projection in the needle that narrows the passage of the anchor.
FIG 59 is a cross-sectional view of the distal end portion of the suture instrument that has a projection in the needle that narrows the passage of the anchor.
FIG 60 is a drawing that describes the operation of pushing out the first anchor.
FIG 61 is a drawing that describes the operation of pushing out the second anchor.
FIG 62 is a perspective view of the distal end portion of the suture instrument that has a crank-shaped side hole.
FIG. 63 is a drawing that describes the linking structure between the second anchor and the pusher.
FIG 64 is a drawing that describes the operation of pushing out the first anchor.
FIG 65 is a drawing that describes the operation of rotating to push out the second anchor.
FIG 66 is a drawing that describes the operation of pushing out the second anchor.
FIG. 67 is a cross-sectional view of the distal end portion of the suture instrument that houses two anchors of differing lengths.
FIG 68 is a drawing that describes the operation of pushing out the first anchor.
FIG. 69 is a drawing that describes the operation of pushing out the second anchor.
FIG 70 is a drawing that shows a pusher that has two recessed portions that each houses an anchor in different directions.
FIG 71 is a drawing that describes the operation of pushing out the first anchor.
FIG 72 is a drawing that describes the operation of pushing out the second anchor.
FIG 73 is a perspective view of the distal end portion of the suture instrument that has projections in the needle that are capable of engaging with the anchors.
FIG. 74 is a cross-sectional view along the line C-C in FIG 73.
FIG 75 is a cross-sectional view of the distal end portion of the suture instrument shown in FIG 73.
FIG 76 is a drawing that describes the operation of pushing out the first anchor.
FIG 77 is a drawing that describes the operation of pushing out the second anchor.
FIG 78 is a drawing that describes the embodiment of mounting a ring instead of projections.
FIG 79 is a cross-sectional view of when the ring is mounted.
FIG 80 is a drawing that describes the embodiment of attaching a wire spring instead of projections.
FIG 81 is a drawing that shows the distal end portion of the suture instrument that has a narrowed portion of the side hole of the needle.
FIG 82 is a drawing that describes the operation of pushing out the first anchor.
FIG 83 is a drawing that shows the distal end portion of the suture instrument in which the side hole is formed in a cam shape.
FIG 84 is a cross-sectional view of the distal end portion of the suture instrument that has a spring that holds the anchor.
FIG 85 is a drawing that describes the operation of pushing out the first anchor.
FIG 86 is a drawing in which the second anchor is engaged by the spring when the first anchor has been pushed out.
FIG 87 is a perspective view of the distal end portion of the suture instrument that is provided with a housing portion that houses the second anchor.
FIG 88 is a perspective view that shows the second anchor and the housing portion.
FIG. 89 is a cross-sectional view along the line E-E in FIG. 88.
FIG 90 is a cross-sectional view along the line F-F in FIG 88.
FIG 91 is a view that shows in cross section a portion of the distal end portion of the suture instrument that provides in the pusher a hook that engages with the second anchor.
FIG 92 is a view in which the hook is released from the second anchor by pulling the pusher from the state of FIG 91.
FIG 93 is a schematic view that shows the step of observing the outer portion side of the stomach.
FIG 94 is a schematic view that shows the step of thrusting the tissue with the needle of the suture instrument.
FIG 95 is a schematic view that shows the step of pushing out the anchor from the needle to the inner portion side of the stomach.
FIG 96 is a schematic view of both anchors being placed on the outer portion side of the stomach.
FIG 97 is a schematic view that shows the step of tightening the perforation with the suture tool.
FIG 98 is a drawing that shows the step of inserting an endoscope in the stomach of a patient and observing the intended incision line from within the stomach.
FIG 99 is a drawing that shows the step of thrusting the needle at the suture position.
FIG 100 is a drawing that shows the step of thrusting the needle and pushing out an anchor to the side of the abdominal cavity.
FIG 101 is a drawing that shows two suture tools mounted.
FIG 102 is a drawing that shows the example of two suture instruments respectively being passed through two channels.
FIG. 103 is a drawing that shows the step of making an incision along the intended incision line.
FIG 104 is a drawing that shows the step of inserting the endoscope insertion part from a perforation into an abdominal cavity and performing a procedure.
FIG 105 is a drawing in which forceps are passed through the endoscope in order to tighten the suture tool.
FIG 106 is a drawing in which the suture thread of the suture tool is gripped by the forceps.
FIG 107 is a drawing in which the suture tool is tightened by the outer sheath of the forceps.
FIG 108 is a drawing in which the perforation is sutured by tightening two suture tools.
FIG 109 is a drawing for describing the order of tightening a plurality of suture tools.
FIG 110 is a drawing that describes the step of closing the perforation around the endoscope.
FIG 111 is a drawing that describes the step of closing the perforation around the endoscope.
FIG 112 is a drawing that describes the step of closing the perforation around the overtube.
FIG 113 is a drawing that describes the step of closing the perforation around the overtube.
FIG 114 is a drawing for describing a method of suturing down the wall of a hollow organ to reduce the diameter thereof.
FIG 115 is a drawing that describes by the cross section along G-G in FIG 114 the process of the hollow organ diameter becoming smaller by suturing down the wall of a hollow organ.

### [BRIEF DESCRIPTION OF THE REFERENCE NUMERALS]

11, 101, 101A, 101B, 101C, 101D, 131, 131A, 131B, 141, 151, 161, 171, 181, 201, 211, 231, 241 suture instrument
13 inner sheath (elongated member)
14, 115, 132, 140, 145, 152, 162, 172, 182, 203, 212, 232, 242 needle
15, 104, 137, 154, 164, 174, 184, 204, 252 side hole
16 suture tool
20, 134, 135, 142A, 142B, 144, 145, 148A, 148B pusher
21 distal end portion (push-out control portion)
25 suture thread
27, 103, 120, 133, 156A, 156B, 165A, 165B, 175, 185A, 185B, 206A, 206B, 216A, 216B, 237A, 237B, 244A, 244B, 244C anchor
116, 153, 163, 173, 183, 203, 213, 234, 243 opening portion
32A, 33A, 33B slanted face
106 plate spring (push-out control portion)
110 erecting hook (push-out control portion)
117 projection (push-out control portion)
141A, 141B, 146A, 146B lumen
176 recessed portion
177 engagement portion
209A projection portion (push-out control portion)
210A engagement portion (push-out control portion)
215 narrowed portion (push-out control portion)
239 spring (push-out control portion)
245 housing portion (push-out control portion)
261 recessed portion
262 hook (push-out control portion)

### [BEST MODE FOR CARRYING OUT THE INVENTION]

Next, embodiments and examples useful for understanding the invention shall be described with reference to the drawings. Note that in the descriptions of each embodiment and example, the same reference numbers shall be given to identical portions. Also, descriptions of overlapping portions shall be omitted.

### [First example]

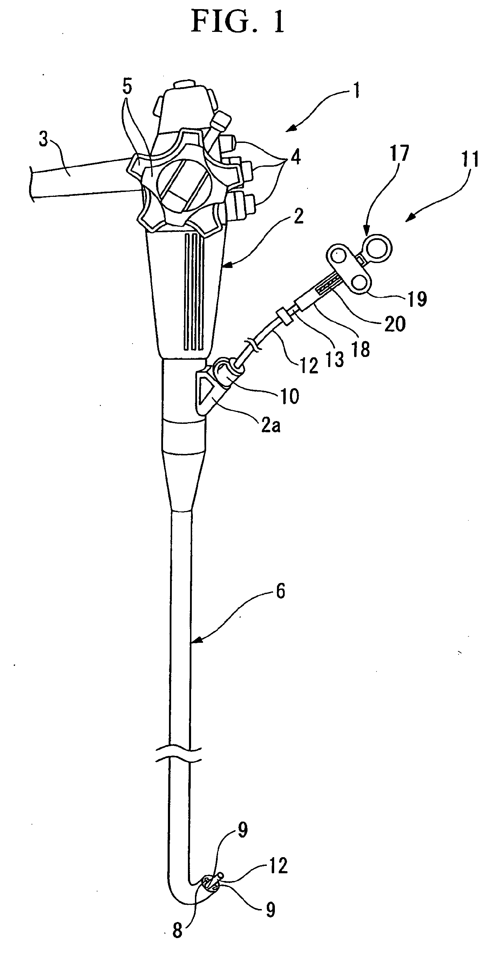

In FIG 1, an endoscope and a suture instrument used in this example useful for understanding the invention are shown. An endoscope 1 (flexible endoscope) has an endoscope operation unit 2 which is operated by an operator. The endoscope operation unit 2 is connected to a control device via a universal cable 3 and equipped with various switches 4 and angle knobs 5. At the distal end of the endoscope operation unit 2, an endoscope insertion part 6 that is flexible and long is extendedly formed. At the distal end of the endoscope insertion part 6, an observation device 7 which obtains an image of the internal body, a lighting unit 8, and a distal end opening of a channel 9 are provided. As the observation device 7, an image taking device having a CCD (Charge Coupled Device) or an optical fiber can be used. The lighting unit 8 has an optical fiber that conducts light from a light source. The channel 9 opens at a lateral part 2a of the endoscope operation unit 2 through the endoscope insertion part 6. At an opening of the lateral part 2a, a cap 10 is provided. In the cap 10, an insertion hole is formed, and a treatment tool such as a suture instrument 11 is inserted into the channel 9 through this insertion hole. That is, the endoscope 1 or the channel 9 are used as tools for inserting a treatment tool such as the suture instrument 11 from a natural opening of a living body into a hollow organ.

As shown in FIGS. 1 to 3, in the suture instrument 11, a flexible inner sheath 13 is passed through the inside of a flexible outer sheath 12 so as to be able to freely move forward or backward. To the distal end (remote end when viewed from the hand side) of the inner sheath 13 (elongated member), a hollow needle 14 is fixed. The needle 14 is blocked and the distal end thereof has a sharp end portion. A suture tool 16 is contained inside of the needle 14. Two anchors 27 of the suture tool 16 are housed in the needle 14. Each of the lengths of the outer sheath 12 and the inner sheath 13 is longer than that of the channel 9 of the endoscope 1. At a base end (proximal end viewed from the hand side) of the inner sheath 13, an operation unit 17 is provided. The operation unit 17 has a handle 19 which can freely slide with respect to a main body 18 of the operation unit. To the handle 19, a base end (proximal end) of a pusher 20 is fixed. The pusher 20 extends through the inside of the inner sheath 13 to the inside of the needle 14. A distal end portion 21 at the remote side of the pusher 20 is pressed against the suture tool 16.

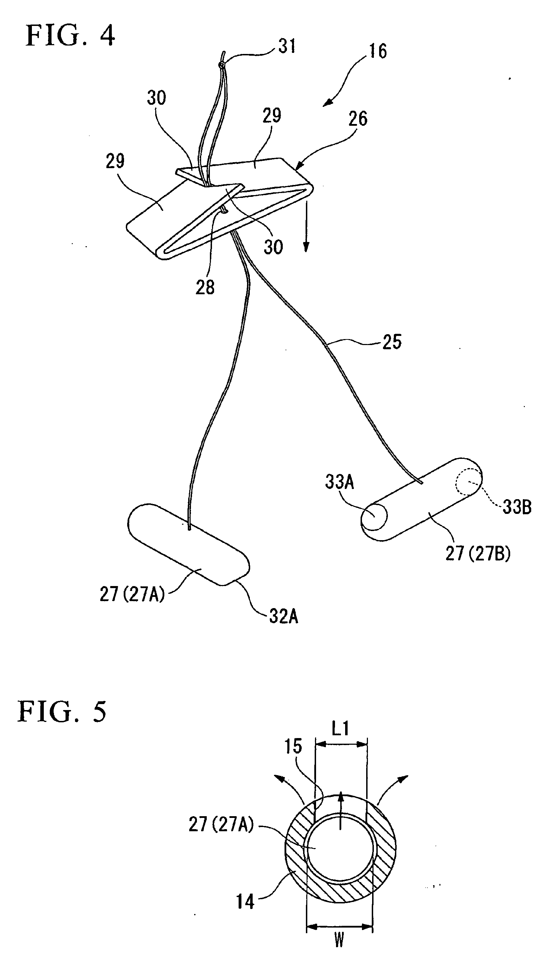

As shown in FIG 4, the suture tool 16 has a suture thread 25. The suture thread 25 is folded approximately in two and a knot 31 is formed in the vicinity of its turn-around point. Moreover, the suture thread 25 is bundled at both end portions thereof and passed through a stopper 26 that is substantially triangular. To each end portion of the suture thread 25, an anchor 27 is fixed one by one. The anchor 27 has a cylindrical, elongated shape and the suture thread 25 is fixed at the approximately center portion in a longitudinal direction of the anchor 27. The anchors 27 consist of a first anchor 27A which has a slanted face 32A formed at one end portion and a second anchor 27B with slanted faces 33A, 33B at both end portions. Two slanted surfaces are formed so as to be approximately parallel at both of the slanted faces 33A, 33B of the second anchor 27B. The stopper 26 includes an elongated plate member in which a hole 28 is formed at the center portion in a longitudinal direction thereof, through which the suture thread 25 is passed. Both end portions 29 in a longitudinal direction of the stopper 26 are diagonally folded back to hold the suture thread 25 therebetween. Both end portions 29 in a longitudinal direction of the stopper 26 are cut to form triangular sections 30. Both end portions 29 of the stopper 26 are diagonally folded back so that the sections 30 intersect with each other to hold the suture thread 25 therebetween. As a result, the suture thread 25 is prevented from passing through a space formed between end portions 29. When the knot 31 of the suture thread 25 is pulled in a direction away from the stopper 26, both end portions 29 of the stopper 26 are slightly opened. Accordingly, the stopper 26 allows the suture thread 25 to move in this direction. On the other hand, when end portions of the suture thread 25 at the side of the anchors 27 are pulled, the suture thread 25 is ready to move in a direction shown by an arrow in FIG 4. However, both end portions 29 of the stopper 26 close and secure the suture thread 25 at this time, and thereby the suture thread 25 does not move.

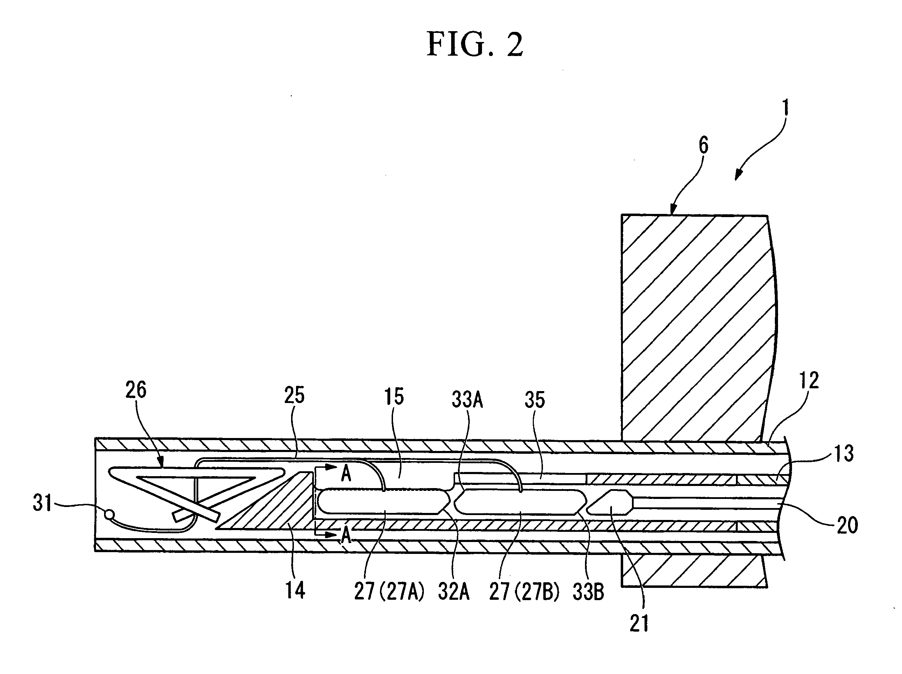

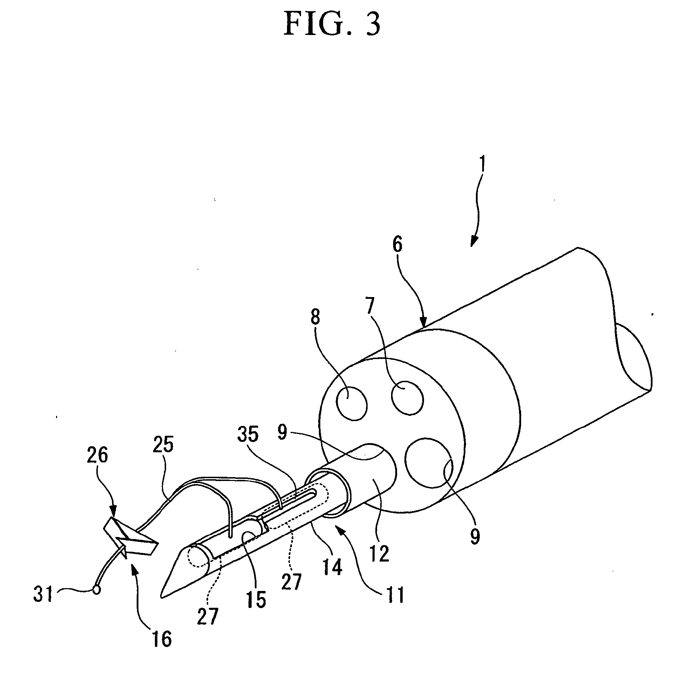

As shown in FIG 3, the two anchors 27 of the suture tool 16 are sequentially held from the distal end side in an inner hole of the needle 14 in the order of the first anchor 27A and the second anchor 27B. A side hole 15 for releasing the anchors 27 is provided in the side portion of the needle 14. The side hole 15 is provided at the distal end side of the needle 14, and extends in the length direction of the needle 14. The length thereof is approximately equal to the length of the anchor 27. As shown in FIG 5, a width L1 that perpendicularly intersects the length direction of the side hole 15 is less then the diameter W of the anchor 27. As shown in FIG 2 and FIG 3, a side hole 35 with a decreased width is further provided in an extending manner from the base end side of the side hole 15. The suture thread 25 of the second anchor 27B is passed through this side hole 35. The slanted face 32A of the first anchor 27A is faced to the opposite side of the position of the side hole 15. The second anchor 27B is positioned so that the slanted face 33A at one end portion faces the slanted face 32A of the first anchor 27A, that is, faces the side hole 15. At this time, the slanted face 33B at the other end portion of the second anchor 27B is faced to the opposite side of the side hole 35. The distal end portion 21 of the pusher 20 is a push out control portion that slopes so as to make contact facing the slanted face 33B having this orientation.

As shown in FIG. 2, the stopper 26 is held at a more distal end than the needle 14 in the outer sheath 12. The number of the anchors 27 and the shape of the stopper 26 are not limited to the example shown in the figures.

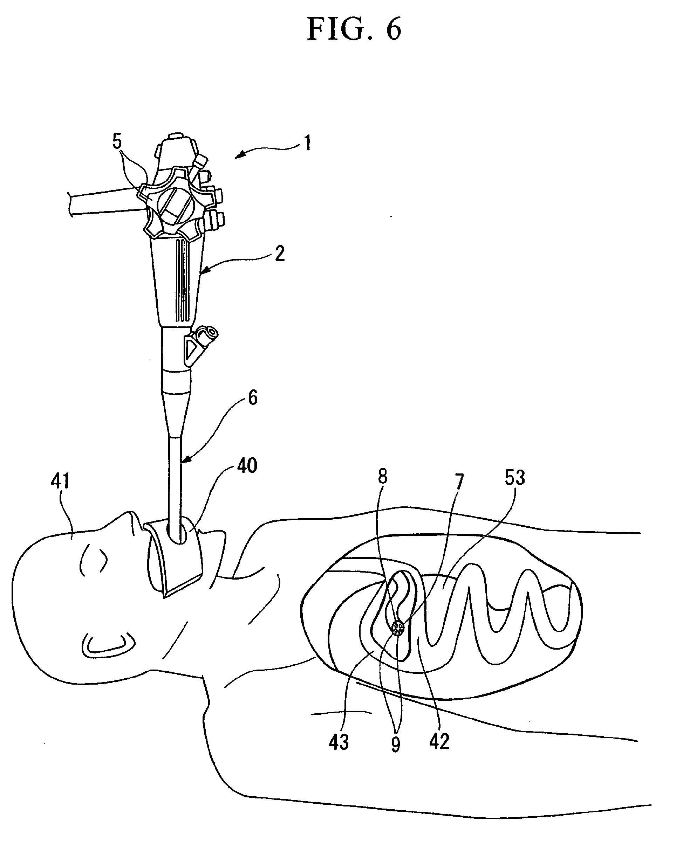

Next, a suturing method of this example will be explained mainly with reference to FIGS. 6 to 13. FIGS. 6 to 13 are pattern diagrams illustrating procedure and show the stomach as an example of a hollow organ.

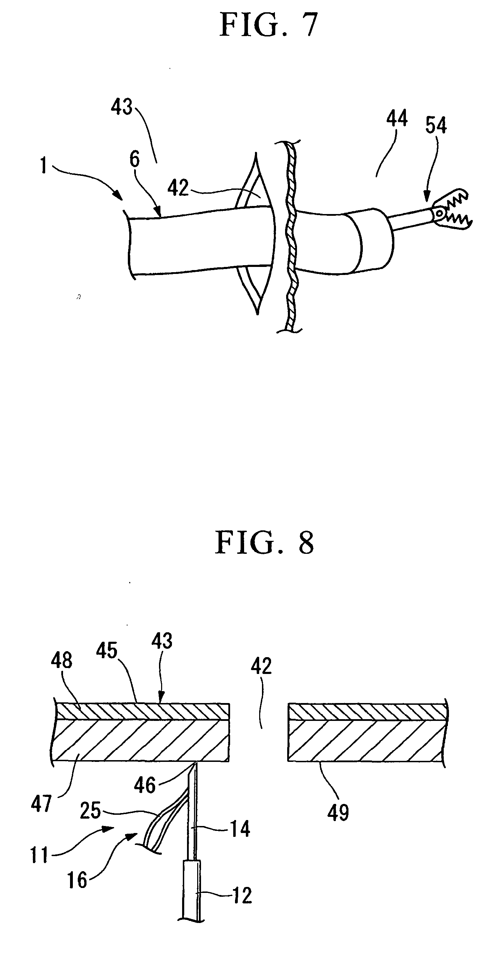

As shown in FIG 6, the endoscope insertion part 6 is inserted from the mouth of a patient 41 prepared with a mouthpiece 40, and the distal end of the endoscope insertion part 6 is made to bend by the angle knob 5. A needle-shaped knife, which is a high-frequency cutting tool, is passed through the channel 9 of the endoscope insertion part 6, to form a perforation in the wall portion of the stomach 43. As shown in FIG. 7, the endoscope insertion part 6 is led through the perforation 42 into the abdominal cavity 44. The forceps 54 are passed through the channel 9, and the treatment of the abdominal cavity 44 is performed by the forceps 54.

After the treatment is completed, the suture instrument 11 is passed through the channel 9 in the place of the forceps 54. The distal end portion of the endoscope insertion part 6 is bent, and the distal end opening of the channel 9 is faced toward a wall portion 45 of the stomach 43 in the vicinity of the perforation 42 from the outside of the stomach 43 (abdominal cavity 44 side), and the needle 14 of the suture instrument 11 is projected from the outer sheath 12. The stopper 26 falls to the abdominal cavity 44 side. The first anchor 27A at this stage does not drop from the needle 14 because the opening width of the side hole 15 of the needle 14 is narrow. As shown in FIG 8, the needle 14 is thrust at a puncture position 46 at a predetermined distance from the opening periphery of the perforation 42. After the needle 14 has passed through the wall portion 45 of the stomach 43 in the order of a muscle layer 47 and a membrane 48, the pusher 20 is moved forward (refer to FIG 2).

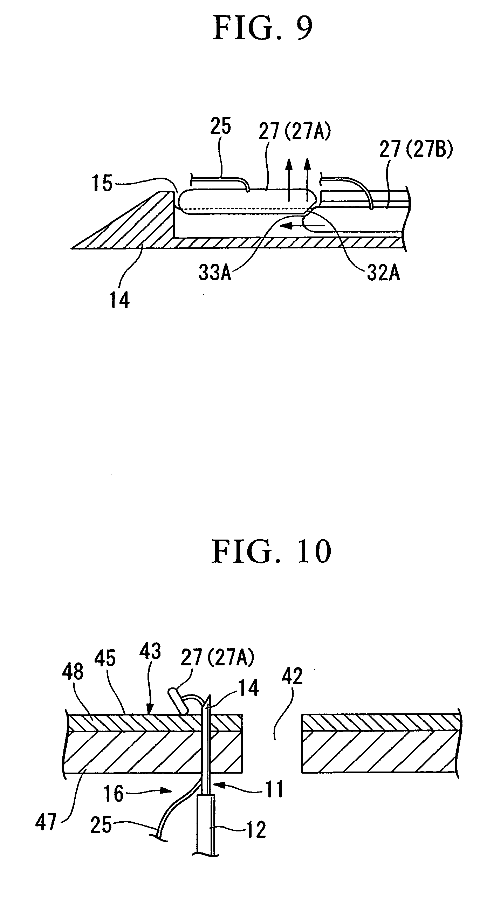

The slanted portion of the distal end portion 21 of the pusher 20 pushes the slanted face 33B of the second anchor 27B. The second anchor 27B advances, and pushes the first anchor 27A, which makes surface contact via the slated faces 32A and 33B, toward the distal end of the needle 14. Here, the contact surfaces of the anchors 27A, 27B are slanted toward a direction intersecting the lengthwise direction of the needle 14, and slant toward the side of the side hole 15. Moreover, the distal end portion of the needle 14 is blocked. Accordingly, as shown in FIG 9, the movement direction of the first anchor 27A is the direction from the length direction of the needle 14 to the side hole 15. The first anchor 27A is released from the needle 14 while spreading the opening portion of the side hole 15 in the direction shown by the arrows in FIG 5. With the release of the first anchor 27A, the second anchor 27B moves to the vacated space. However, after the release of the first anchor 27A, the opening width of the side hole 15 reverts to the original size, so the second anchor 27B does not drop from the needle 14.

As shown in FIG 10, after the first anchor 27A is pushed out, the needle 14 is drawn out from the wall portion 45. The first anchor 27A remains on the inner portion of the stomach 43, and because the suture thread 25 penetrates through the wall portion 45, is drawn to the outside of the stomach 43.

Similarly, a position that is symmetrical with respect to the puncture position to sandwich the perforation 42 therebetween is set as a puncture position 49 (see FIG. 8), and the needle 14 is thrust again at this puncture position 49. When the needle 14 penetrates through the wall portion 45, the second anchor 27B is pushed out from the needle 14 by the pusher 20. Since the contact surface between the pusher 20 and the second anchor 27B is slanted facing the side hole 15, the second anchor 27B, after abutting the blocked end portion of the needle 14, moves toward the side hole 15, and while spreading the opening width of the side hole 15, is released to outside of the needle 14.

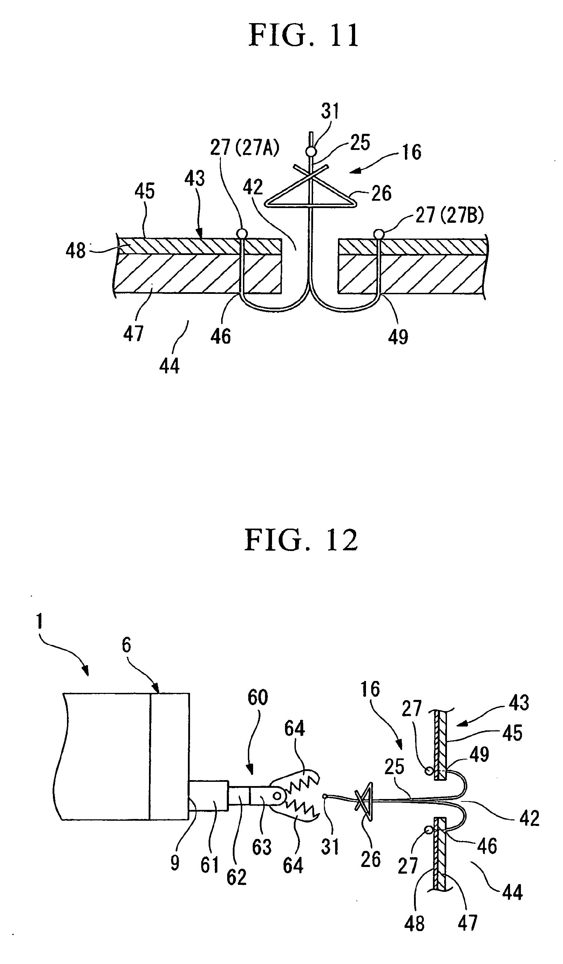

Thereafter, when the needle 14 is drawn out, as shown in FIG 11, the suture tool 16 is mounted in the vicinity of the perforation 42.

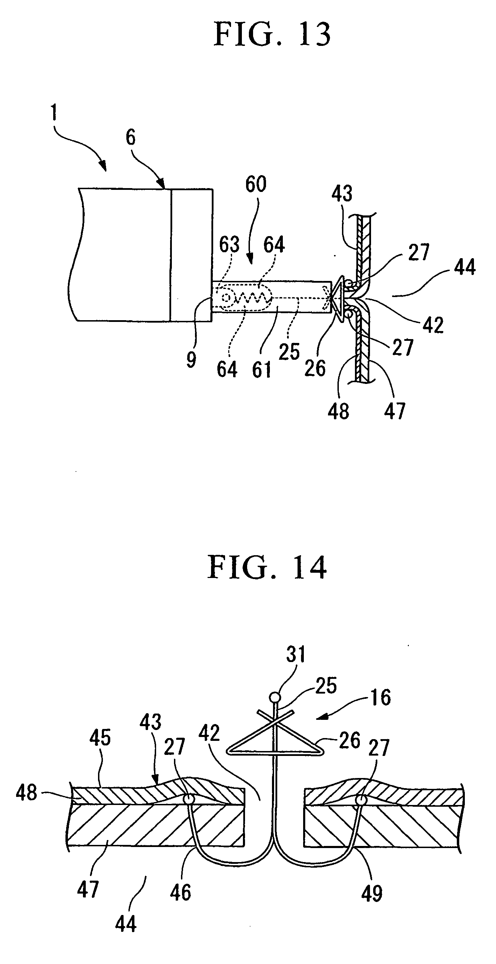

Next, as shown in FIG 12, the endoscope insertion part 6 is drawn back to the inside of the stomach 43, and the suture tool 16 is tightened by the forceps 60. The forceps 60 has an outer sheath 61 having an external diameter larger than the anchor 27, and an inner sheath 62 passed through the outer sheath 61 so as to freely move forward or backward. At the distal end of the inner sheath 62, a supporting member 63 is provided, and a pair of grip segments 64 is supported on the supporting member 63 so as to freely open or close. The knot 31 of the suture thread 25 of the suture tool 16 is gripped by the grip segments 64. When the outer sheath 61 is moved forward, the distal end of the outer sheath 61 presses against the stopper 26. As shown in FIG. 13, when the outer sheath 61 moves further forward, the stopper 26 is pushed into the wall portion 45. Since the stopper 26 is constructed to be able to move in this direction, the stopper 26 moves toward the wall portion 45. Since the position of the pair of the grip segments 64 does not change, the stopper 26 moves relatively forward with respect to the suture thread 25. As a result, the distance between the stopper 26 and the anchor 27 decreases, and the suture thread 25 is cinched.

After suturing of the perforation 42 is completed, the outer sheath 61 is moved backward, and the grip segments 64 are then opened. The suture thread 25 leaves the grip segments 64. Although the end portion of the stopper 26 can move in a direction in which the tissue is tightened up by the suture thread 25, it acts to tighten up the suture thread 25 in a direction for loosening the suture thread 25. As a result, the suture thread 25 maintains a sutured state without loosening, even if the suture tool 16 is placed inside of the stomach 43.

In this example, the endoscope 1 and the operation channel 9 are used as tools for passing the needle 14. The needle 14 that is inserted from a natural opening of a living body is thrust from the side of the abdominal cavity 44 into the wall portion 45, and since the suture thread 25 is tightened up so that the puncture positions 46, 49 are approximately matched, the muscle layers 47 can be held together. In the example, it is possible to tightly close the perforation 42 by more reliably holding the muscle layers 47 together. Therefore, the process of adhesion is fast, and recovery is quick.

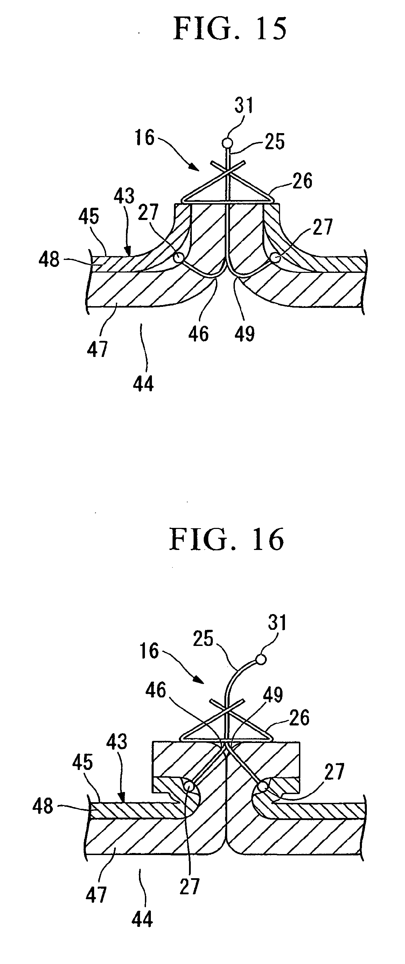

FIGS. 14 to 16 show modified examples. As shown in FIG 14, the needle 14 is passed from the puncture positions 46, 49 through the muscle layer 47, and the anchors 27 are placed between the muscle layer 47 and the membrane 48. The suture thread 25 thus passes through only the muscle layer 47 to be mounted on the wall portion 45. As shown in FIG 15, when the suture thread 25 is tightened, the puncture positions 46, 49 approach each other, the tissue is wound into the inside of the stomach 43, and the outer surfaces of the muscle layer 47 make close contact. As shown in FIG 16, when the puncture positions 46, 49 are approximately matched, the perforation 42 is closed. Since the anchors 27 are placed between the muscle layer 47 and the membrane 48, in order to insert the suture thread 25, leaks in the abdominal cavity 44 from the inside of the stomach 43 along the holes that are formed in the wall portion 45 are prevented. Also, since the anchors 27 are not exposed to the inside of the stomach 43, the anchors 27 are protected from stomach acid. Moreover, since the anchors 27 are not exposed to the inside of the stomach 43, it is possible to maintain the anchors 27 in an approximately germ-free state. In this case, recovery is promoted.

### [Second example]

A second example useful for understanding the invention shall be described with reference to the drawings. Note that descriptions of constituent elements and actions that are the same as those of the first example shall be omitted.

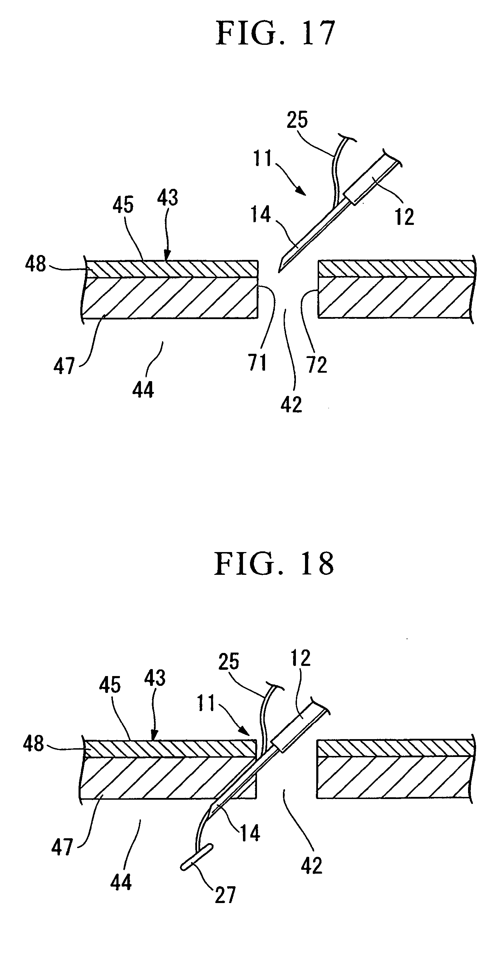

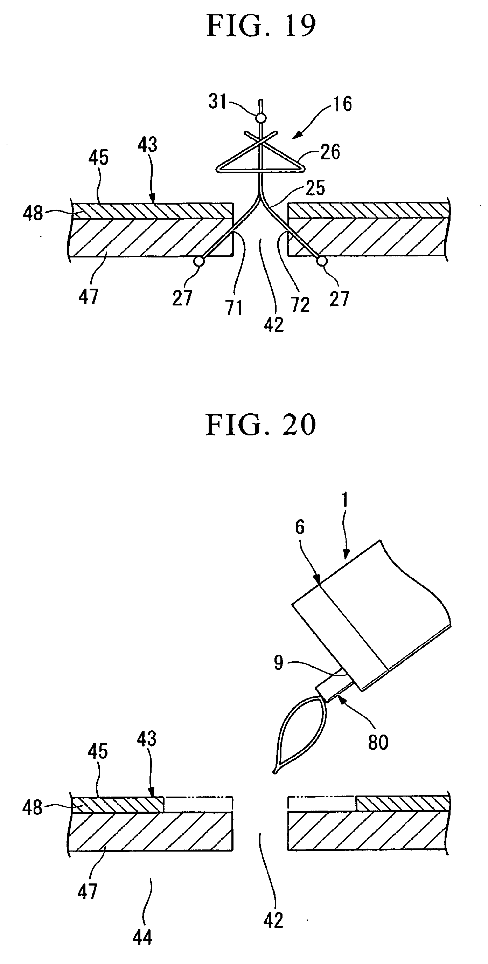

As shown in FIG 17, a suture instrument 11 is passed through a channel of an endoscope insertion part 6. The needle 14 is thrust obliquely at a predetermined puncture position 71 in the muscle layer 47 that is exposed to the inner periphery face of the perforation 42. As shown in FIG 18, the needle 14 that is thrust from the puncture position 71 obliquely penetrates through the muscle layer, and when the distal end portion of the needle 14 is exposed on the abdominal cavity 44 side, the first anchor 27 is pushed out to the abdominal cavity 44 side. The needle 14 is then removed, and obliquely thrust at a predetermined puncture position 72 in the muscle layer 47 that is exposed to the inner periphery on the opposite side of the perforation 42 shown in FIG. 17. It is preferable that the puncture position 71 and the puncture position 72 are at approximately identical positions in the thickness direction of the wall portion 45. When the needle 14 has obliquely penetrated through the muscle layer 47 from the inner periphery face of the perforation 42 to the abdominal cavity 44 side, the second anchor 27 is pushed out to the abdominal cavity 44 side. Thereafter, when the needle 14 is removed from the wall portion 45, as shown in FIG 19, the second anchor 27 is placed on the abdominal cavity 44 side similarly to the first anchor 27. The suture thread 25, after obliquely piercing the muscle layer 47, is pulled into the stomach 43 along the inside of the perforation 42.

In this example, after the needle 14 is obliquely thrust into the muscle layer 47 that is exposed to the inner periphery of the perforation 42, and the anchors 27 are placed on the abdominal cavity 44 side, the tissue is fastened together by the suture thread 25 so that puncture positions 71, 72 coincide. Therefore, it is possible to reliably hold the muscle layers 47 together. Accordingly, the muscle layers 47 are reliably adhered, and it is possible to quickly close the perforation 42. Holes that allow passage of the suture thread 25 are not formed in the membrane.

### [Third example]

A third example useful for understanding the invention shall be described with reference to the drawings. Note that descriptions of constituent elements and actions that are the same as those of the first example shall be omitted.

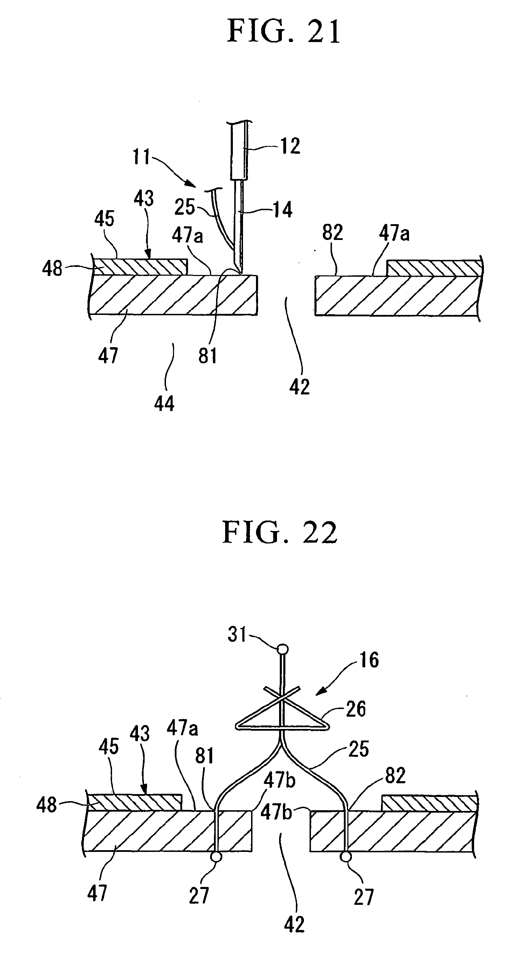

As shown in FIG 20, a treatment tool 80 such as a snare is passed through a channel 9 of an endoscope insertion part 6, and the membrane 48 around the perforation 42 is resected. After the membrane 48 is resected, the suture tool 16 is mounted by the suture instrument 11. As shown in FIG 21, the needle 14 is thrust at a predetermined position (puncture position 81) in the inner surface 47a of the muscle layer 47 that is exposed by resection of the membrane 48. When the needle 14 has penetrated through the muscle layer 47 and protrudes on the abdominal cavity 44 side, the anchor 27 is pushed out. When the needle 14 is drawn out from the muscle layer 47, the suture thread 25 passes through the muscle layer 47, and the anchor 27 is placed on the abdominal cavity 44 side. Similarly, the needle 14 is thrust again at an approximately symmetrical position to sandwich the perforation 42 therebetween. A puncture position 82 at this time is set at a location at which the inner surface 47a of the muscle layer 47 is exposed as a result of resection of the membrane 48. The needle 14 penetrates through the muscle layer 47 from the inner surface side (inner portion side of the stomach 43) to the outer surface side (outer portion side of the stomach 43), and the second anchor 27 is pushed out. As shown in FIG 22, when the needle 14 is drawn out, the suture tool 16 is mounted so as to straddle the perforation 42.

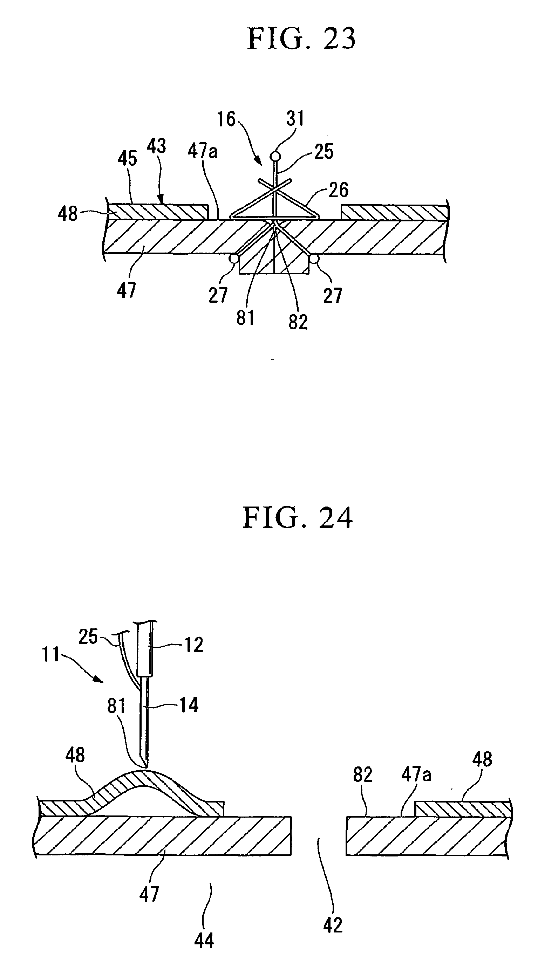

This suture tool 16 is fastened similarly to the second example. By pulling the suture threads 25, the inner periphery faces of the perforation 42 are drawn in and made to stick together. The suture thread 25 is drawn until the puncture positions 81, 82 approximately coincide. At this time, the end portions 47b of the muscle layer 47 are initially abutted together on the inner portion side of the stomach 43, and with this as a starting point, the inner faces 47a are pulled together. As shown in FIG. 23, the tissue is pushed out so that the inner periphery surfaces of the perforation 42 face the outer portion side of the stomach 43, and by resection of the membrane 48, the exposed inner faces 47a of the muscle layer 47 are stuck together.

### [Fourth example]

A fourth example useful for understanding the invention shall be described with reference to the drawings. Note that descriptions of constituent elements and actions that are the same as those of the first example shall be omitted.

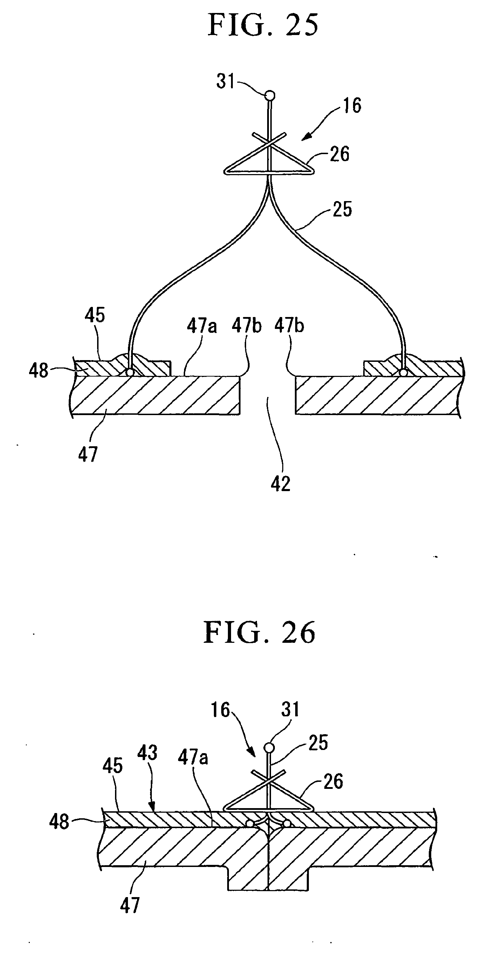

As shown in FIG. 24, for example a treatment tool such as a snare is passed through the channel 9 of an endoscope insertion part 6, and the membrane 48 around the perforation 42 is resected. After the membrane 48 is resected, the suture tool 16 is mounted by the suture instrument 11. The needle 14 is thrust at a portion that is made to swell by a local injection into the membrane 48. When the needle 14 is thrust into the membrane, the anchor 27 is pushed out. When the needle 14 is drawn out from the membrane, the suture thread 25 penetrates through the membrane, and the anchor 27 is placed on the abdominal cavity 44 side. Similarly, the needle 14 is thrust again at an approximately symmetrical position to sandwich the perforation 42 therebetween. The puncture position at this time is a portion that is made to swell by a local injection. The membrane is penetrated with the needle 14, and the second anchor 27 is pushed out. As shown in FIG 25, when the needle 14 is drawn out, the suture tool 16 is mounted so as to straddle the perforation 42.

This suture tool 16 is fastened similarly to the second example. By pulling the suture threads 25, the inner periphery faces of the perforation 42 are drawn in and made to stick together as shown in FIG. 26.

### [Fifth example]

A fifth example useful for understanding the invention shall be described with reference to the drawings. Note that descriptions of constituent elements and actions that are the same as those of the first example shall be omitted.

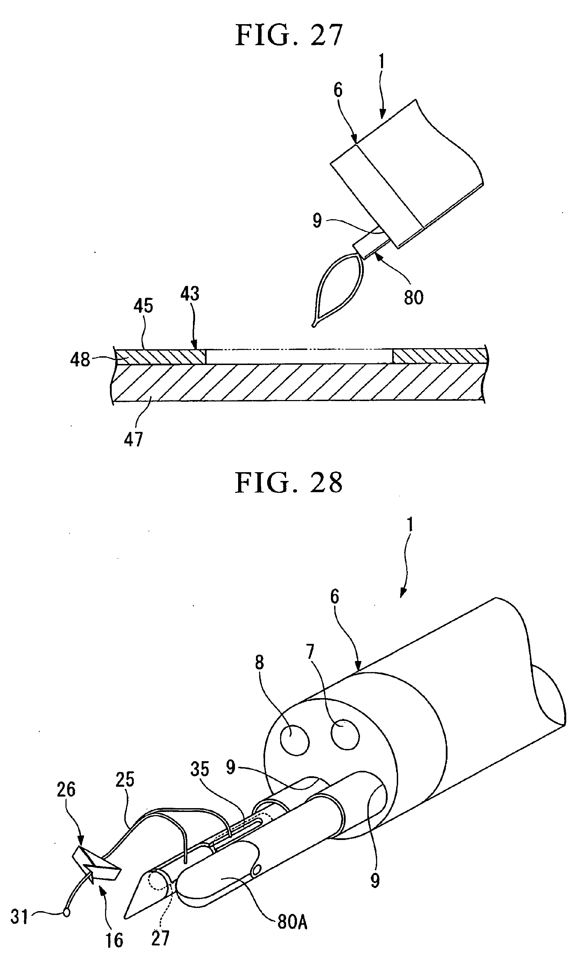

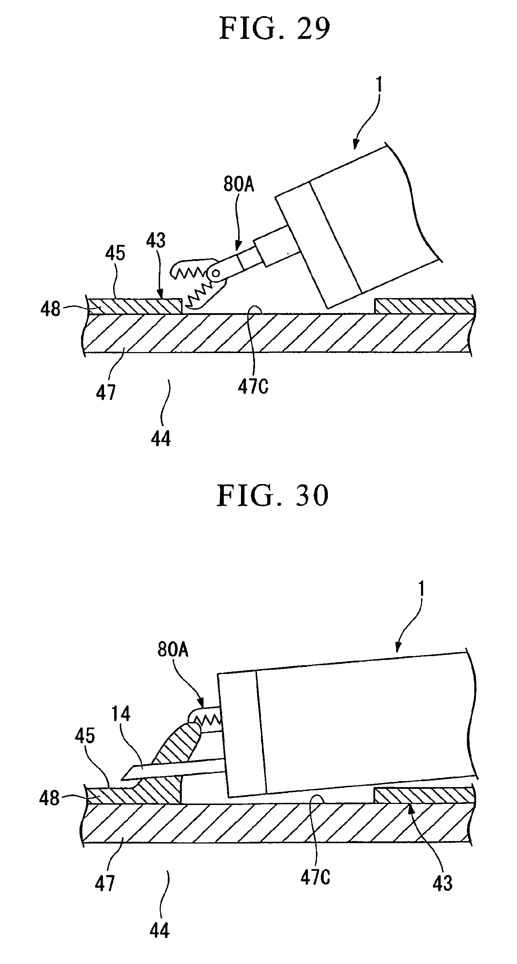

As shown in FIG. 27, for example a treatment tool such as a snare is passed through the channel 9 of an endoscope insertion part 6, and the membrane 48 is resected. After the membrane 48 is resected, the suture tool 16 is mounted by the suture instrument 11. Also, as shown in FIG. 28, a grasping forceps 80A is passed through a separate channel 9. As shown in FIG 29, an end of the membrane 48 that is resected is grasped by the grasping forceps 80A. As shown in FIG 30, after penetrating the needle 14 through the membrane 48 that is raised by the grasping forceps 80A, the first anchor 27A is pushed out. When the needle 14 is drawn out from the membrane, the suture thread 25 penetrates through the membrane, and the anchor 27 is placed on the abdominal cavity 44 side. Similarly, the needle 14 is thrust again at an approximately symmetrical position to sandwich a membrane resected surface 47c therebetween. The membrane is penetrated with the needle 14, and the second anchor 27B is pushed out. When the needle 14 is removed, the suture tool 16 is mounted so as to cover the membrane resected surface 47c.

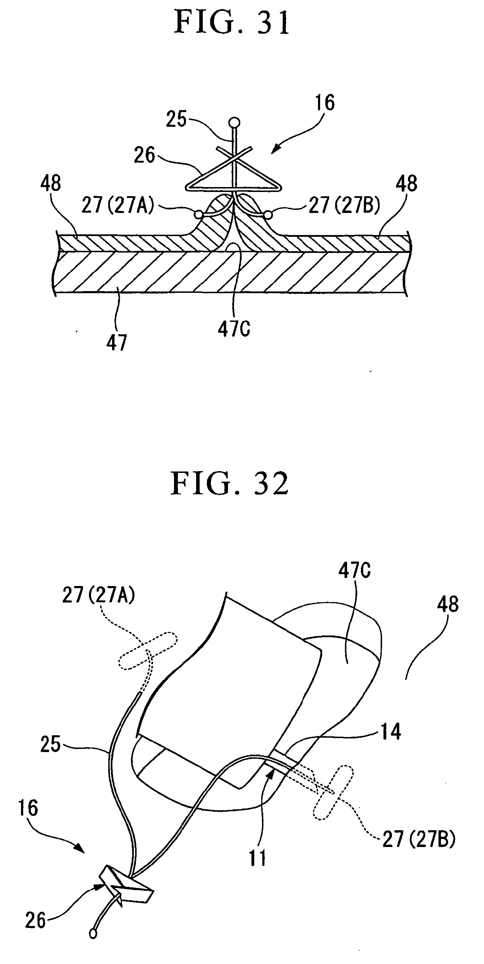

This suture tool 16 is fastened similarly to the second example. By pulling the suture threads 25, the membranes around the membrane resected surface 47c are drawn in and made to stick together as shown in FIG 31.

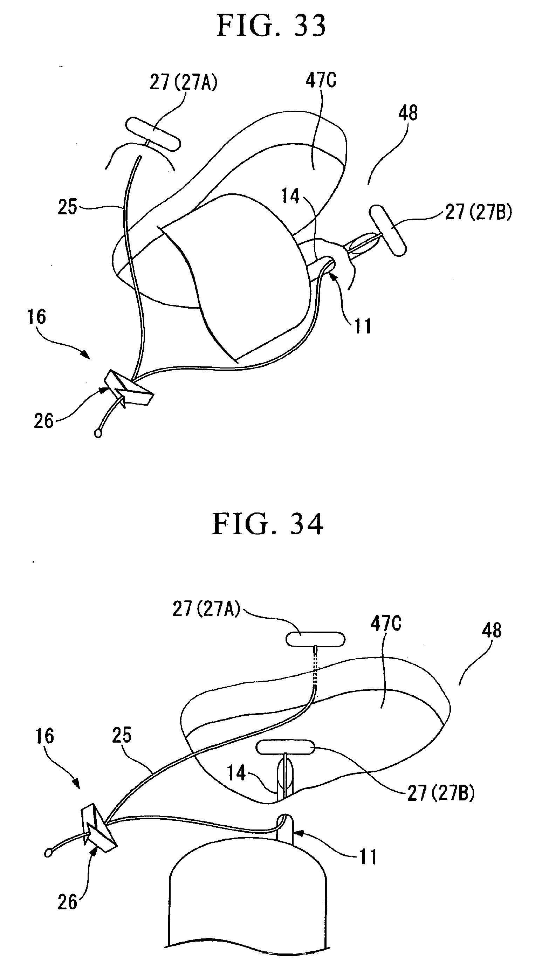

Here, various modifications of the placement state of the anchors 27 and method of inserting the anchors 27 are possible as shown in FIG. 32 to FIG 34. Also, the insertion method is not particularly limited. Note that FIG 32 shows a modification in which, by thrusting the needle 14 from the inner side to the outer side of the perforation, the anchors 27 are placed on the outer side. FIG. 33 shows a modification in which the needle is thrust in tissue that is swollen on the inner side, and the anchors are placed on the inner side. FIG 34 shows a modification in which one of the anchors 27 is placed by being passed from the outer side to the inner side, while the other anchor 27 is placed by being passed from the inner side to the outer side.

### [Sixth example]

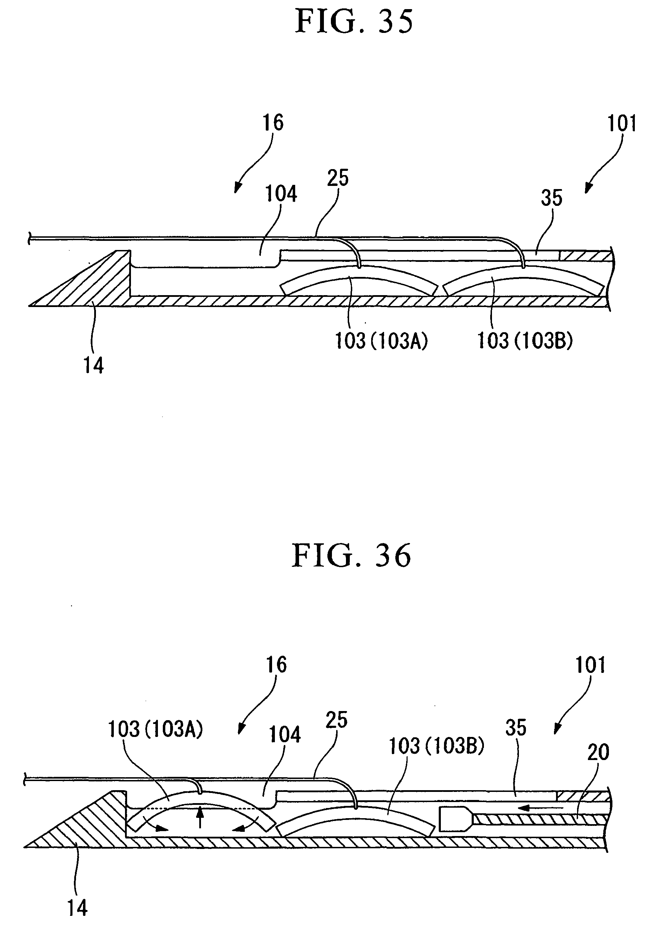

As shown in FIG 35, the needle 14 of the suture tool 101 is provided at the distal end of the inner sheath 13 that is an elongated member (also in the examples below unless particularly stated). The needle 14 has a hollow shape with a sharp distal end portion that is closed, and two anchors 103 of the suture tool 16 are housed therein, being arranged along the lengthwise direction. The end of a suture thread 25 is passed through the center portion of each anchor 103. The anchors 103 smoothly curve from the center portion to both ends in the lengthwise direction to form an arch shape that becomes convex toward a side hole 15. The anchor 103 is made of an elastically deformable material, and is biased in the flat direction when housed in the inner hole of the needle 14. Since the anchors 103 in the needle 14 are compressed in a direction that perpendicularly intersects the lengthwise direction (vertical direction), the dimension in this direction decreases, and the dimension in the lengthwise direction is extended. The diameter of the inner hole of the needle 14 is less than the height of the anchors 103 from the side portion to the center portion when restored to their original shape (natural shape) without the application of an external force. Note that the anchors 103 may be formed by flexing a cylindrical member into an arch shape, or flexing a plate member into an arch shape.

A side hole 104 that is continuous with the inner hole is formed at the side portion of the distal end side of the needle 14. The side hole 104 extends in the lengthwise direction of the needle 14, and the length thereof is shorter than the length of the anchor 103 that is biased in the flat direction in the needle 14. The opening width that perpendicularly intersects the lengthwise direction of the side hole 104 is preferably greater than the width of the anchor 103. However, in the case of widening the opening width by elastically deforming the needle 14, the opening width may be less than or equal to the width of the anchor 103.

When pushing out a first anchor 103A from the needle 14, a pusher 20 pushes a second anchor 103B to the distal end. When the first anchor 103A moves to the formation position of the side hole 104, that is, directly under the side hole 104, the bias of the first anchor 103A in the flat direction is released, and so the first anchor 103A reverts to its natural shape. Therefore, since the height from the end portion to the center portion of the anchor as shown in FIG. 36 exceeds the inner diameter of the needle 14, and the length of the first anchor 103A becomes less than or equal to the length of the side hole 104, the anchor 103A is released from the needle 14 through the side hole 104.

Also, when it is desired to more reliably push out the first anchor 103A from the needle 14, the first anchor 103B is pushed further to the distal end side by the pusher 20. The first anchor 103A that is pushed by the second anchor 103B flexes because the distal end portion of the needle 14 is blocked. Due to the compression of the first anchor 103A in the lengthwise direction, the height from the end portion to the center portion of the anchor further increases, and the first anchor 103A is pushed out from the needle 14 through the side hole 104.

In this suture tool 101, since pushing out of the anchors 103 is controlled by utilizing the elastic deformation of the anchors 103, it is possible to reliably push out the anchors 103 one at a time from the needle 14.

Note that in this suture tool 101, when the anchors 103 have moved to the formation position of the side hole 104, the anchors 103 are released from the needle 14 by utilizing the elastic deformation of the anchors 103 and the push-bending by the pusher 20. However, the anchors 103 may be released from the needle 14 using only the elastic deformation of the anchors 103. Also, the anchors 103 may be released by using only the push-bending by the pusher 20 without using the elastic deformation of the anchors 103. The second anchor 103B advances into the space that is vacated when the first anchor 103A has been pushed out. Since the second anchor 103B at this time is longer than the side hole 104, it does not drop out from the side hole 104. To push out the second anchor 103B, by pushing with the pusher 20, the second anchor 103B is compressed in the direction of the side hole 104 to be made the same as or less than the length of the side hole 104. When the pusher 20 is further advanced, the second anchor 103B is pushed out from the side hole 104.

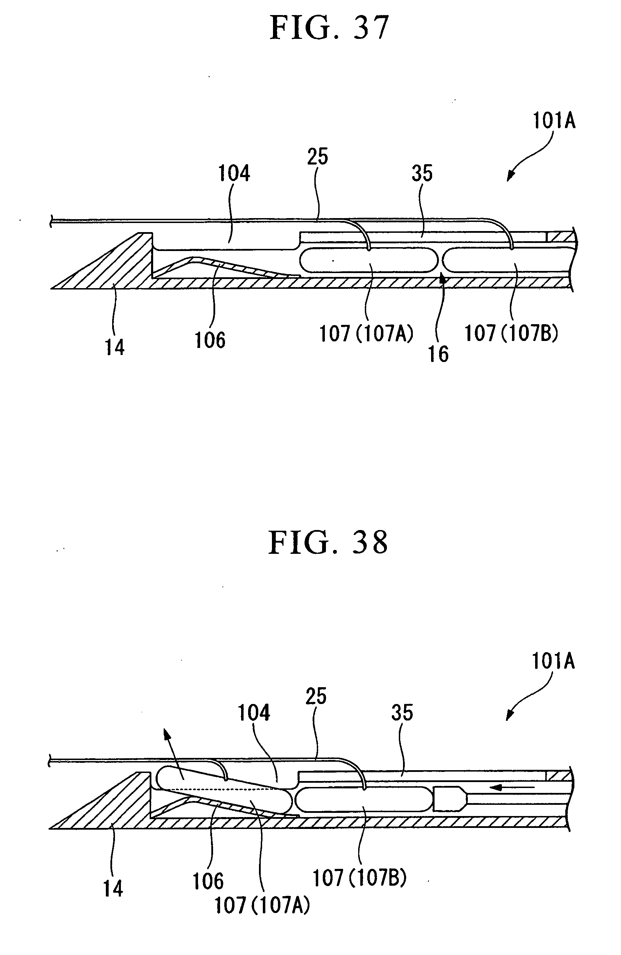

Here, FIGS. 37 to 45 show modified examples of this example.

In the needle 14 of a suture tool 101A shown in FIG 37, a plate spring 106 is provided as a push-out control portion at the distal end side of the needle 14 in conjunction with the formation position of the side hole 104. The plate spring 106 is disposed with a slope so that the distal end side protrudes toward the side hole 104. The two anchors 107 of the suture tool 16 have a cylindrical, elongated shape, with the suture thread 25 extending from the center portion. In this initial state, the two anchors 107 are disposed more to the base end side than the plate spring 106. As shown in FIG 38, when the pusher 20 moves forward, the first anchor 107A is moved to the distal end side of the needle 14 via the second anchor 107B. The first anchor 107A moves forward while pushing down the plate spring 106, but when the first anchor 107A runs onto the plate spring 106, it is pushed toward the side hole 104 by the restoring force of the plate spring 106, and pushed out from the side hole 104. Once the plate spring 106 has been restored, it works to prevent the second anchor 107B from jumping out. To push out the second anchor 107B, the second anchor 107B is similarly made to run onto the plate spring 106 while pushing it down, and then pushed out. Thus in this suture tool 101A, the same effect as above is obtained by providing the plate spring 106 in the needle 14 as a push-out control portion.

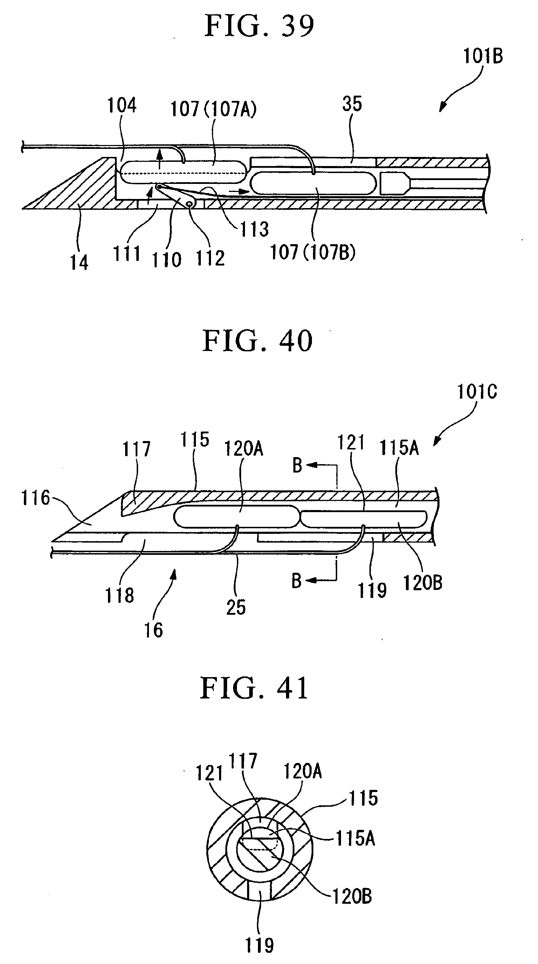

In a suture tool 101B shown in FIG 39, an erecting hook 110 is provided at approximately the opposite side of the formation position of the side hole 104 of the needle 14. The erecting hook 110 is supported to freely rotate on a pin 112 that is attached to a hole 111 that is provided in the needle 14. The distal end portion of the erecting hook 110 is disposed facing the distal end side of the needle 14, and an operation wire 113 is attached here. The operation wire 113 is drawn out to the hand side through the inside of the needle 14. When pushing out the first anchor 107A, the first anchor 107A is moved to the formation position of the side hole 104, that is, onto the top of the erecting hook 110, by pushing the second anchor 107B toward the distal end with the pusher 20. When the erecting hook 110 is made to rise toward the side hole 104 by pulling the operation wire 113, the first anchor 107A is pushed by the erecting hook 110 and pushed out from the needle 14. When the first anchor 107A has been pushed out, the operation wire 113 is pushed to house the erecting hook 110 in the hole 111. In this state, even if the second anchor 107B moves to the formation position of the side hole 104, it does not move in the direction of the side hole 104, and so the second anchor 107B is not pushed out from the needle 14. By providing the erecting hook 110 as a push-out control portion in the needle 14 in this suture tool 101 B, the same effect as above is obtained.

A suture tool 101C shown in FIG 40 has a hollow needle 115. The needle 115 has an opening portion 116 that slopes obliquely toward the distal end. A projection 117 is protrudingly provided at the inner circumferential portion of the base end side of an opening portion 116. As shown in FIG 41, the projection amount of the projection 117 is less than the radius of the inner hole 115A of the needle 115, and the base end side of the projection 117 is a smooth curve. Also, a side hole 118 is formed at a position on the opposite side of the projection 117. A side hole 119 that passes the suture thread 25 is continuously provided at the base end side of the side hole 118.

Elongated anchors of the suture tool 16 consists of a first anchor 120A that is housed at the distal end side of the needle 14 and a second anchor 120B that is housed at the base end side. The outer diameter of the first anchor 120A is greater than the portion that is reduced by the projection 117, but the first anchor 120A is capable of getting out from the side hole 118. The second anchor 120B has a flank face 121 that is cut to allow passage through the projection 117. The length of the second anchor 120B is preferably longer than the length of the side hole 118.

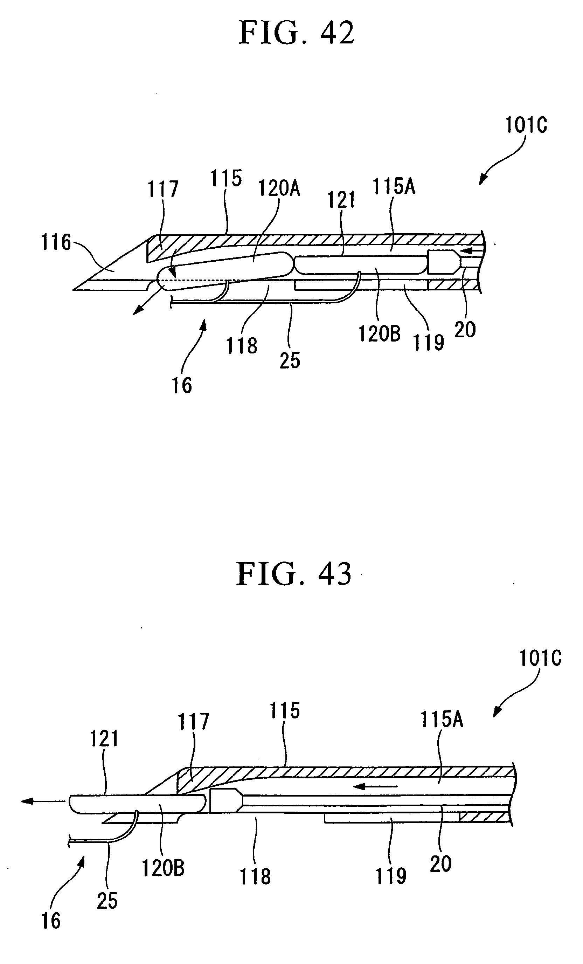

When pushing out the first anchor 120A, the pusher 20 is moved forward. The first anchor 120A is pushed toward the distal end via the second anchor 120B. As shown in FIG 42, the first anchor 120A is guided by the curve of the projection 117 to be pushed out from the side hole 118. In this state, the second anchor 120B cannot leave to the outside from the side hole 118. When pushing out the second anchor 120B, the pusher 20 is moved further forward. As shown in FIG 43, the second anchor 120B slips beyond the projection 117 due to the flank face 121 and moves to the distal end, and is pushed out to outside of the needle 115 from the opening portion 116.

In this suture tool 101C, the same effect as above is obtained by making a difference between the route for pushing out the first anchor 120A by providing the projection 117 as a push-out control portion in the distal end side of the needle 14, and a route for pushing out the second anchor 120B.

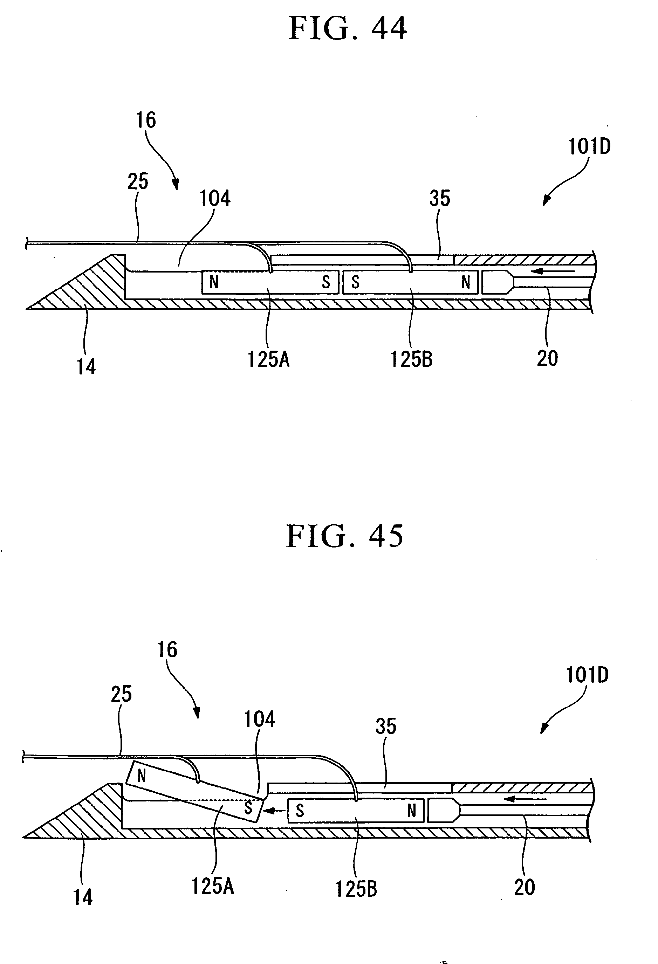

In the suture tool 101 D shown in FIG. 44, two anchors 125A, 125B are manufactured from permanent magnets. The first anchor 125A on the distal end side and the second anchor 125B on the base end side are housed in the needle 14 so that the same magnetic poles face each other. In the example shown in FIG 44, the S-poles face each other. When the pusher 20 is moved forward, the first anchor 125A is pushed out from the needle 14 as shown in FIG 45 by utilizing the repulsion between the permanent magnets. Since the repulsion is utilized, the second anchor 125B is not pushed out simultaneously. In this suture tool 101D, by using permanent magnets for the anchors 125A and 125B, the same effect as above is obtained. Note that the permanent magnets may only be formed at the end portions of the two anchors 125A, 125B that approach each other. Also, both end portions of the two anchors 125A, 125B may be permanent magnets.

### [Seventh example]

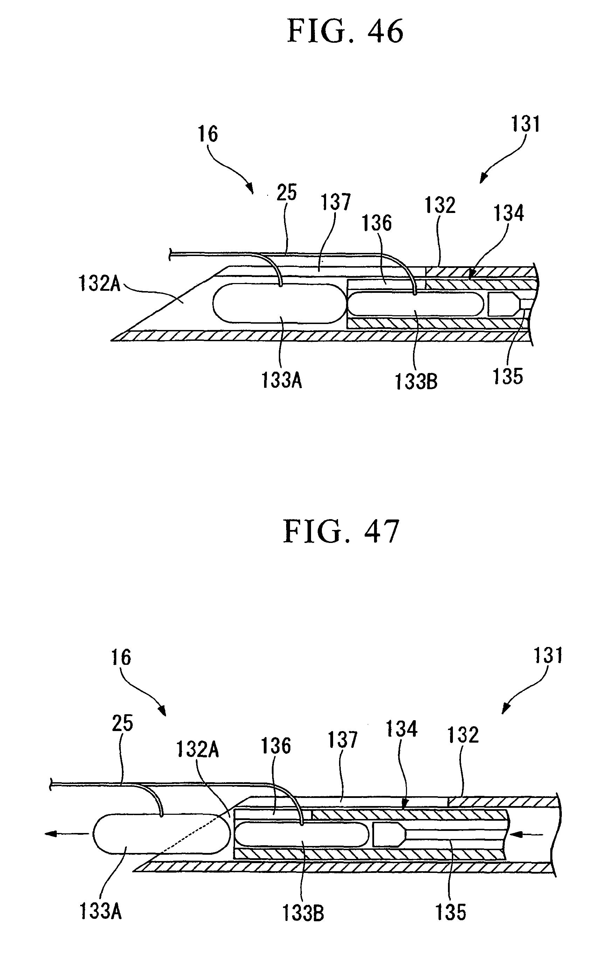

As shown in FIG. 46, a suture tool 131 has a needle 132 that serves as an elongated member. The needle 132 has a hollow shape in which an opening portion 132A is obliquely formed at the distal end. In the inner portion of the needle 132, two anchors 133A, 133B of the suture tool 16, a first pusher 134 that pushes out the first anchor 133A, and a second pusher 135 that pushes out the second anchor 133B are housed. The first anchor 133A is disposed at the distal end side and has an elongated shape with a larger diameter than the second anchor 133B. The second anchor 133B has an elongated shaped that is housed in the cylindrical first pusher 134. A slit 136 is formed in the side portion of the distal end side of the first pusher 134, and the suture thread 25 is drawn out from here. The suture thread 25 is further drawn out to the outside from a side hole 137 of the needle 15. The side hole 137 passes the suture thread 25, but has an opening width that does not allow passage of the anchors 133A, 133B. The second pusher 135 is passed to freely move forward or backward in the first pusher 134. The first and second pushers 134, 135 are drawn to the hand side, and are capable of moving forward or backward independently.

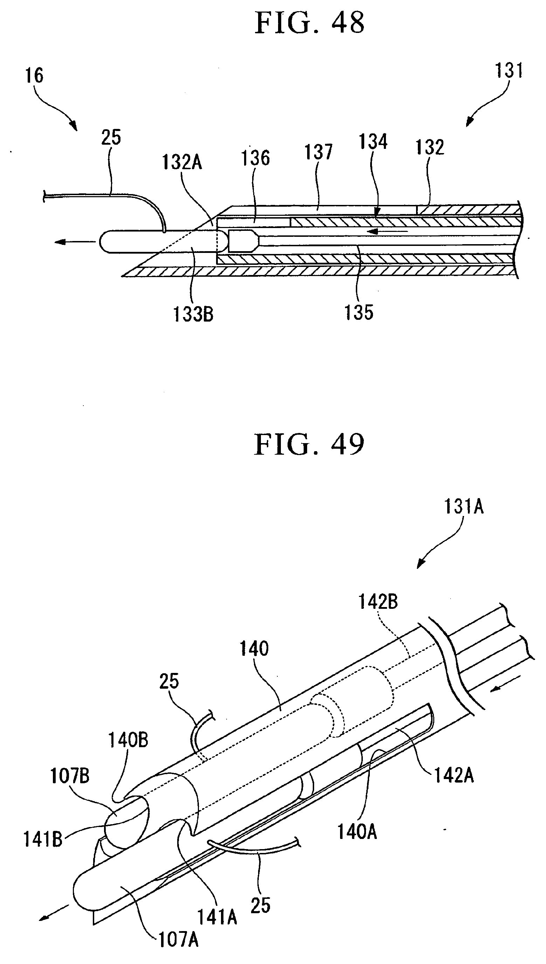

When pushing out the first anchor 133A, the first pusher 134 is moved forward. As shown in FIG. 47, the first anchor 133A is pushed out from the opening portion 132A at the distal end. At this time, the second anchor 133B moves along with the first pusher 134, but since it remains housed in the first pusher 134, it is not released from the needle 132. When pushing out the second anchor 133B, only the second pusher 135 is moved forward. As shown in FIG 48, the second anchor 133B is pushed out from the first pusher 134 and the needle 132.

In this suture tool 131, since a device that pushes out the first anchor 133A and a device that pushes out the second anchor 133B are separately provided, it is possible to reliably push out only the first anchor 133A. Note that the needle 132 may be provided at the distal end of the inner sheath 13.

FIGS. 49 to 50 show modified examples.

In the suture tool 131 A shown in FIG 49, two lumens 141A, 14 1 B are formed in a needle 140. These lumens 141A, 141B are disposed in a direction that perpendicularly intersects the slope direction of the tip of the needle 140. A first anchor 107A and a first pusher 142A are housed in the first lumen 141A, and the suture thread 25 is drawn out from a side hole 140A in the side portion. A second anchor 107B and a second pusher 142B are housed in the second lumen 141B, and the suture thread 25 is drawn out from a side hole 140B in the side portion. When pushing out the first anchor 107A, only the first pusher 142A is moved forward. When pushing out the second anchor 107B, only the second pusher 142B is moved forward. In this suture tool 131A, the two anchors 107A, 107B are housed in different channels, and so by independently providing the pushers 142A, 142B in each channel, it is possible to reliably push out the anchors 107A, 107B one at a time.

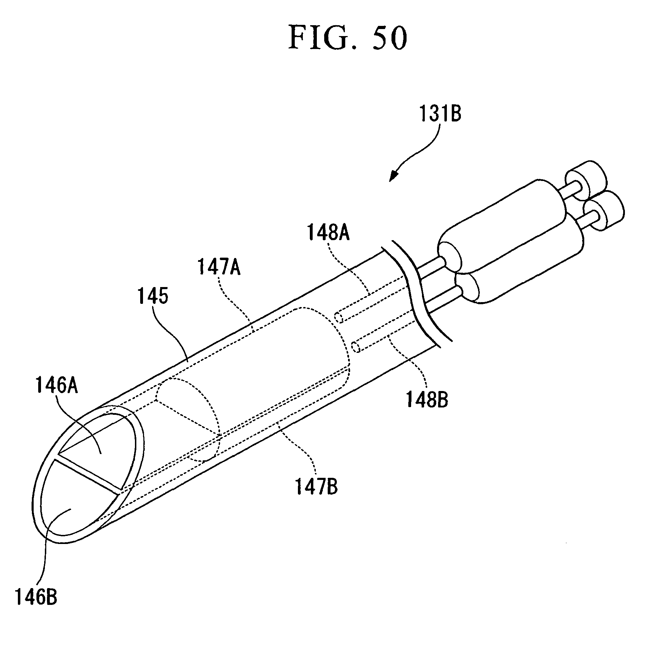

In the suture tool 131B shown in FIG 50, two lumens 146A, 146B are disposed in the needle 145, being arranged along the inclination direction of the tip of the needle 145. The cross-sectional shape of the first lumen 146A is a semicircular shape, with an elongated first anchor 147A and a first pusher 148A being housed therein. The cross-sectional shape of the second lumen 146B is a semicircular shape, with an elongated second anchor 147B and a second pusher 148B being housed therein. The suture threads 25 of each anchor 147A, 147B are drawn out from side holes (not illustrated) formed in the side portion. When pushing out the first anchor 147A, only the first pusher 148A is moved forward. When pushing out the second anchor 147B, only the second pusher 148B is moved forward. In this suture tool 131B, by providing the two pushers 148A, 148B to be independently driven, it is possible to reliably push out the anchors 147A, 147B one at a time. Moreover, since the cross-sectional shape of the lumens 146A, 146B is a semicircular shape, it is possible to make effective use of the space in the needle 145.

### [Eighth example]

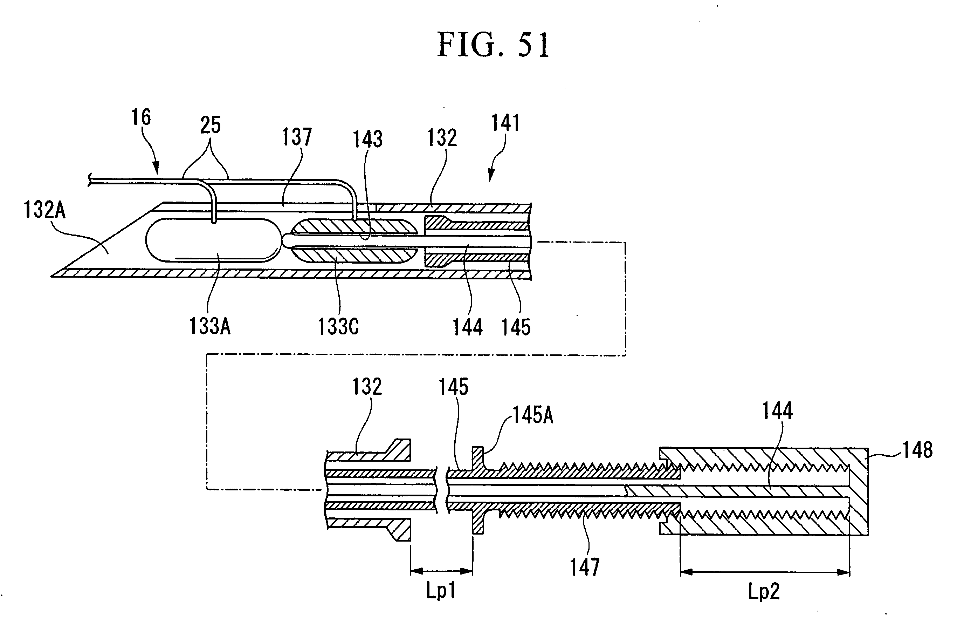

As shown in FIG. 51, an opening portion 132A is obliquely formed at the sharp tip of the distal end of a needle 132 of a suture tool 141, and a first anchor 133A and a second anchor 133C of the suture tool 16 are housed from the distal end side therein. The outer shape of the two anchors 133A, 133C are approximately the same, but a hole 143 that penetrates in the lengthwise direction is formed in the center portion of the second anchor 133C. The first pusher 144 is passed through this hole 143 so as to be able to freely move forward or backward. Moreover, further to the base end side than the second anchor 133C, a second pusher 145 is provided in a cylindrical shape so as to cover the outer circumference of the first pusher 144.

The hand side control unit of the second pusher 145 is drawn out from the base of the sheath 12, and a flange-shaped projection 145A is integrally provided in an extended manner at a position that is drawn out by a predetermined length from the base of the sheath 12. A length Lp1 from the projection 145A to the sheath base end side corresponds to the stroke of the second pusher 145. A male thread 147 is engraved on the outer circumference of the base end side of the second pusher 145 beyond the projection 145A, and a base end portion 148 of the first pusher 144 is screwed onto the thread 147. A distance Lp2 that the second pusher 145 can be screwed into the base end portion 148 of the first pusher 144 becomes the stroke of the first pusher 144 with respect to the second pusher 145. Note that the control unit at hand side of each pusher 144, 145 are not limited to the ones illustrated.

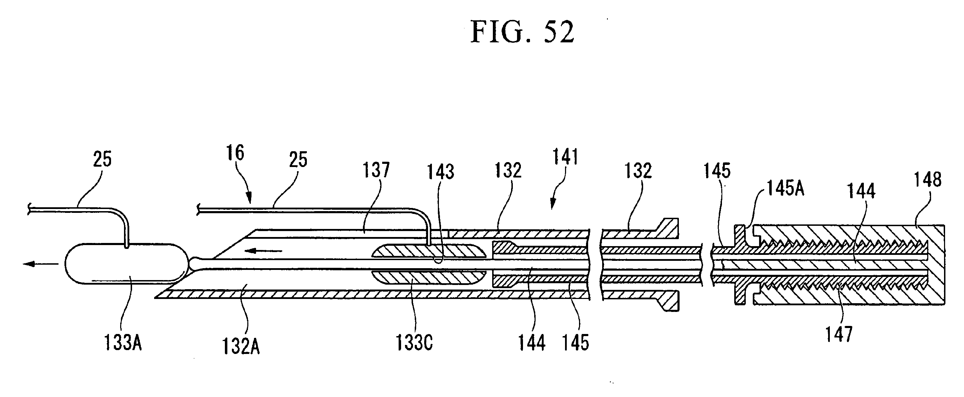

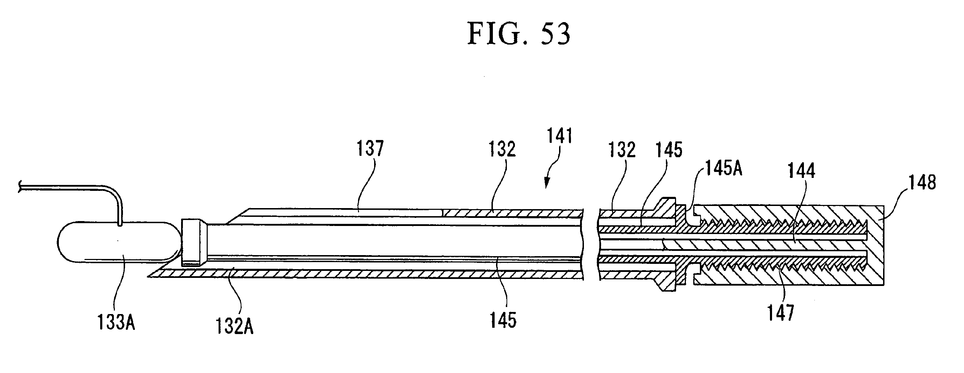

When pushing out the first anchor 133A, the base end portion 148 of the first pusher 144 is rotated without moving the second pusher 145. The first pusher 144 moves forward with respect to the second pusher 145. As shown in FIG. 52, the first anchor 133A is pushed out by the distal end portion of the first pusher 144 that penetrates the second anchor 133C. Since the first pusher 144 does not press the second anchor 133C, the second anchor 133C remains in the needle 132. When pushing out the second anchor 133C, the second pusher 145 is pushed out with respect to the needle 132. As shown in FIG. 53, the projection 145A of the second pusher 145 is made to abut the base end portion of the needle 132. The second anchor 133C is pushed out by the second pusher 145.

In this example, by providing the first pusher 144 that can push out only the first anchor 133A and the second pusher 145 that can push out the second anchor 133C on the same axis, it is possible to reliably push out only the first anchor 133A while reducing the outer diameter. Also, although the second pusher 145 is freely pushed and pulled, the first pusher 144 has screw engagement with the second pusher 145, and the operation for pushing out the anchors 133A, 133B are different with the two pushers 144, 145. Accordingly, it is easy to control the push out of the anchors 133A, 133B.

### [Ninth example]

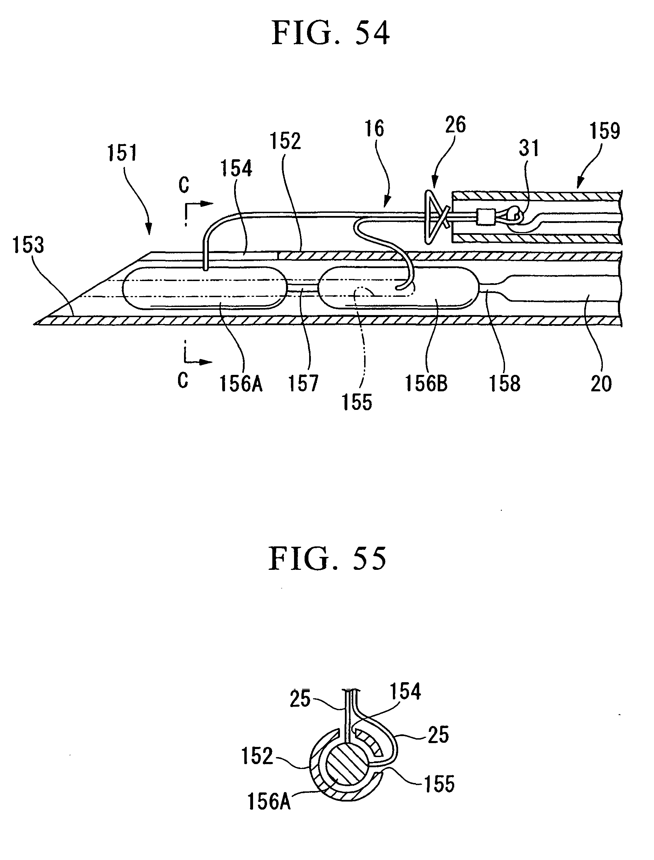

As shown in FIG 54, a needle 152 of a suture instrument 151 has an opening portion 153 that is obliquely formed at the sharp tip of the distal end, with two side holes 154, 155 shifted approximately 90 degrees in the circumferential direction formed in the side portion. Two elongated anchors 156A, 156B arranged in the lengthwise direction are housed in the needle 152. The base portion of the first anchor 156A on the distal end side is connected to the distal end portion of the second anchor 156B by a first connecting portion 157. The base portion of the second anchor 156B is connected to a pusher 20 by a second connecting portion 158. Each of the connecting portions 157, 158 is a push-out control portion that consists of a thin flat member and so can be broken by bending. The orientations of the first connecting portion 157 and the second connecting portion 158 are shifted approximately 90 degrees about the center axis. More preferably, the flat face of the first connecting portion 157 faces the side hole 154, and the flat face of the second connecting portion 158 faces the side hole 155.

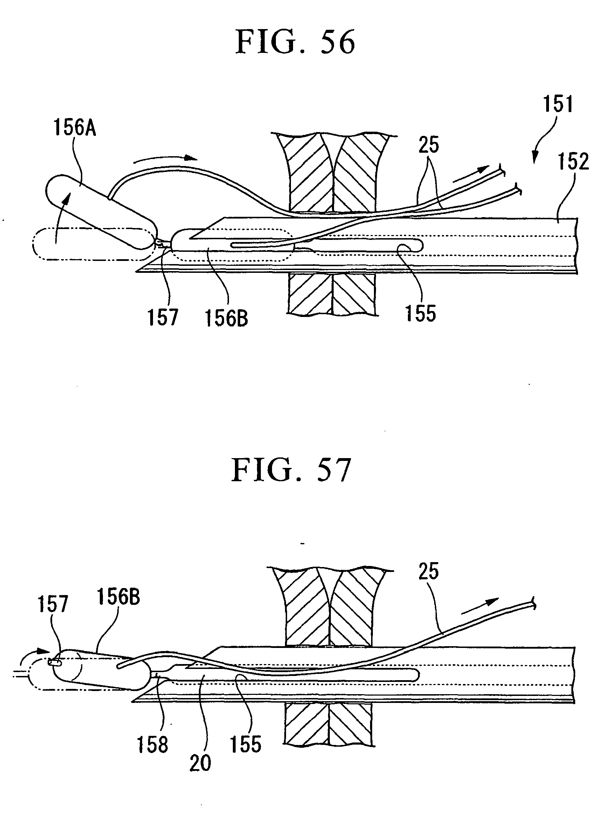

When placing the first anchor 156A, the pusher 20 is moved forward to make the first anchor 156A project out from the tip of the needle 152. Since as the first anchor 156A is unified with the pusher 20, a binding tool 159 that binds the suture tool 16 is pulled back as a whole. As a result, the first anchor 156A is pulled by the suture thread 25. As shown in FIG 56, the first anchor 156A is bent at the first connecting portion 157, and the first connecting portion 157 is broken, so that the first anchor 156A separates from the needle 152 and the second anchor 156B (and the pusher 20). As stated above, when the side hole 154 and the direction of easily bending the first connecting portion 157 agree, it is possible to readily break off the first connecting portion 157. Note that since the pusher 20 and the second anchor 156B are connected via the second connecting portion 158, the second anchor 156B does not detach.

When placing the second anchor 156B, the pusher 20 is moved further forward to cause the second anchor 156B to project from the tip of the needle 152. The binding tool 159 as a whole is pulled back again, causing the second anchor 156B to be pulled by the suture thread 25. As shown in FIG 57, the second anchor 156B is bent at the second connecting portion 158, and the second connecting portion 158 breaks, so that the second anchor 156B separates from the pusher 20. As stated above, when the side hole 155 and the direction of easily bending the second connecting portion 158 agree, it is possible to readily break off the second connecting portion 158.

In this example, the two anchors 156A, 156B that are integrally provided with the pusher 20 are detached in turn. Therefore, it is possible to reliably place only the first anchor 156A.

Also, since the anchors 156A, 156B are formed as one piece, it is possible to lower the anchor manufacturing cost and the suture instrument 151 assembly cost compared to the other examples.

### [Tenth example]

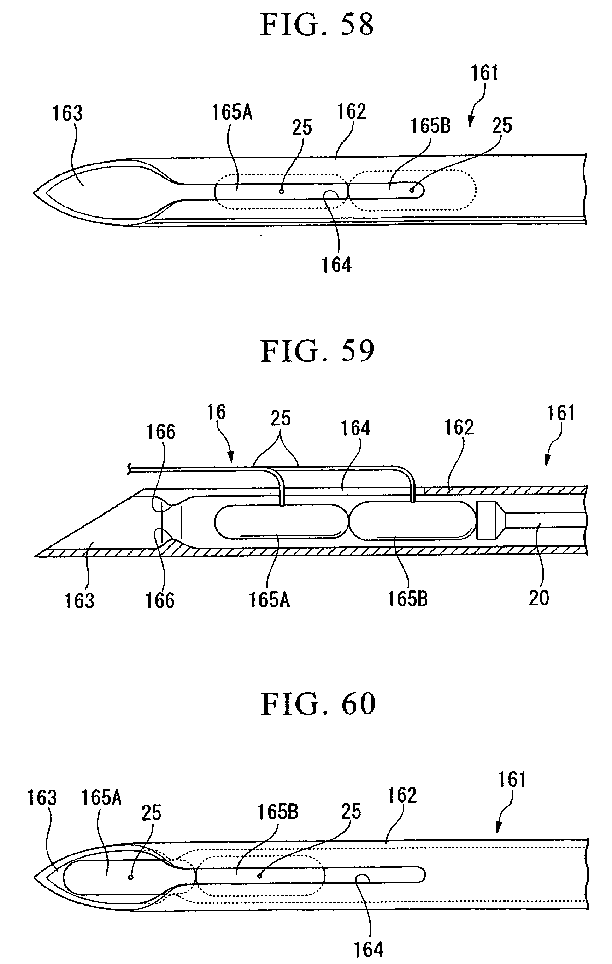

As shown in FIG 58 and FIG 59, an opening portion 163 is obliquely formed at the sharp tip of the distal end of a needle 162 of a suture tool 161, and a side hole 164 is formed along the lengthwise direction of the needle 162 from the base end side of the opening portion 163. The side hole 164 passes the suture thread 25, but cannot pass the anchors 165A, 165B. A projection 166 for projection restriction (push-out control portions) is formed in an approximate ring shape so as to reduce the inner diameter. The projection 166 forms a chamfered smooth curve. The two elongated anchors 165A, 165B are housed in series to the base end side of the projection 166. The outer diameter of the first anchor 165A is less than or equal to the inner circumference of the projection 166. The outer diameter of the second anchor 165B is greater than the first anchor 165A, and greater than the inner circumference of the projection 166.

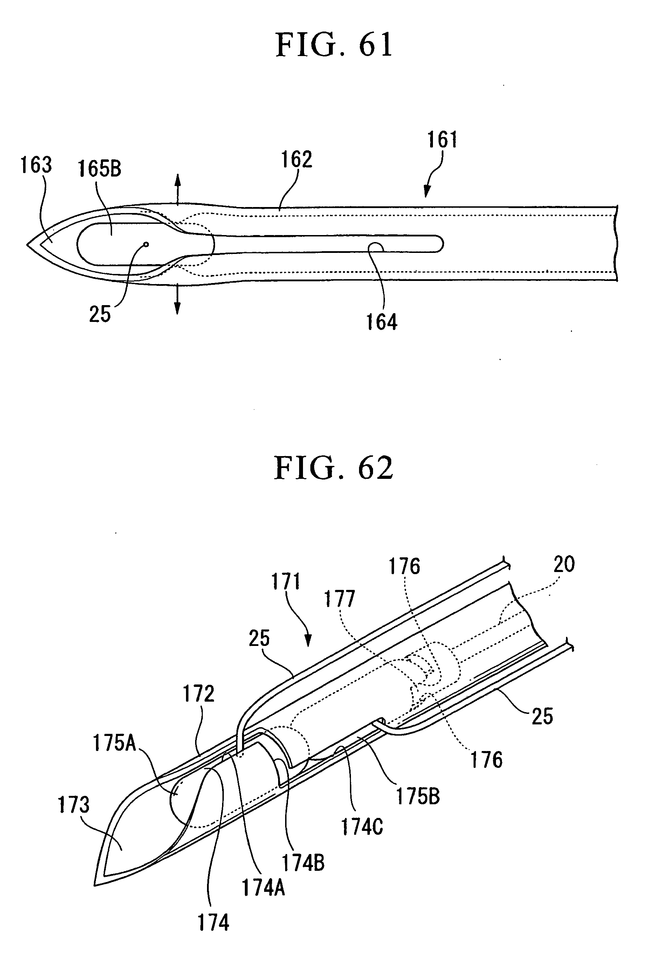

When pushing out the first anchor 165A, the pusher 20 is moved forward. As shown in FIG. 60, the first anchor 165A passes through the projection 166 to be pushed out from the opening portion 163. The second anchor 165B is caught by the projection 166 and stopped. To push out the second anchor 165B, the pusher 20 is pushed further, to apply pressure to the projection 166 with the second anchor 165B, and thereby spread out the distal end portion of the needle 162. As shown in FIG. 61, the space of the formation position of the projection restriction projection 166 expands, and the first anchor 165A is pushed out from the opening portion 163.

In this example, the projection 166 is provided as a push-out control portion in the needle 162, and while the first anchor 165A is pushed out as is, the second anchor 165B cannot be pushed out without applying an even greater force. Therefore, it is possible to reliably push out the anchors 165A, 165B one at a time.

### [Eleventh example]

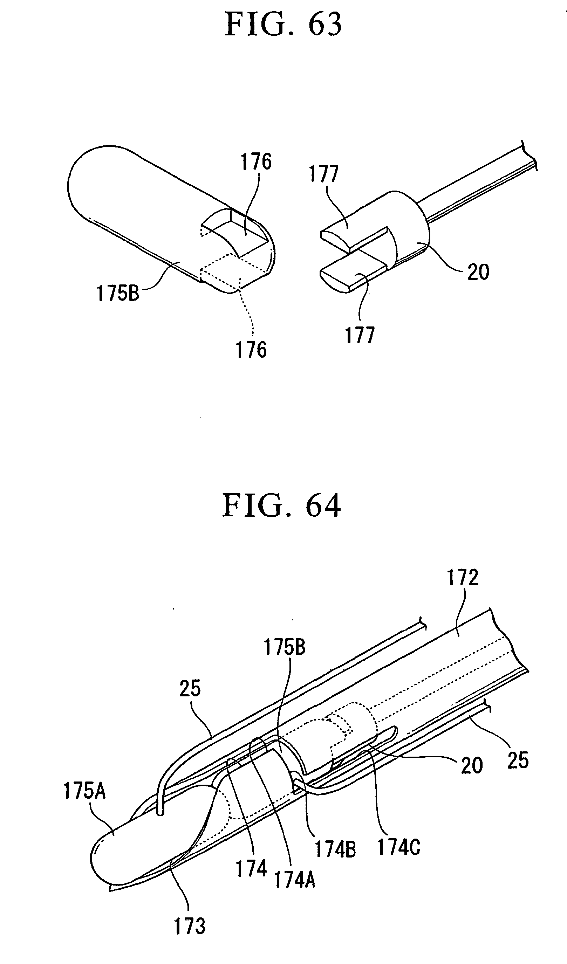

As shown in FIG 62, an opening portion 173 is obliquely formed at the sharp tip of the distal end of a needle 172 of a suture tool 171, and a side hole 174 is formed from the base end side of the opening portion 173. The side hole 174 has a crooked shape consisting of a first portion 174A on the distal end side of the side hole 174 that extends along the lengthwise direction of the needle 172, a second portion 174B that extends in the circumferential direction, and a third portion 174C that extends in the lengthwise direction. Two elongated anchors 175A, 175B of the suture tool 16 are inserted in order in the needle 172. The first anchor 175A is housed near the first portion 174A of the side hole 174, with the suture thread 25 being pulled out from the first portion 174A. The second anchor 175B is housed near the third portion 174C of the side hole 174, with the suture thread 25 being drawn out from the third portion 174C. As shown in FIG. 63, two recessed portions 176 are formed in the base end portion of the second anchor 175B.

Two projection-shaped engagement portions 177 are formed in the distal end of the pusher 20. Each engagement portion 177 has a shape that is capable of engaging with the recessed portion 176 of the second anchor 175B. Thereby, the second anchor 175 and the pusher 20 can be engaged in the rotation direction about the axial line.

To push out the first anchor 175A, the pusher 20 is pushed out. As shown in FIG 64, with the suture thread 25 guided by the first portion 174A of the side hole 174, the first anchor 175 is pushed out from the opening portion 173. Since the suture thread 25 of the second anchor 175B moves along the third portion 174C of the side hole 174 through which it is passed, the second anchor 175B is not pushed out from the needle 172. Note that the third portion 174C is of a length such that when the first anchor 175A has been pushed out, the suture thread 25 of the second anchor remains in the third portion 174C. More preferably, by allowing for the suture thread 25 to move until the connecting portion with the second portion 174B at the distal end of the third portion 174C, it is possible to quickly push out the second anchor 175B with the subsequent operation.

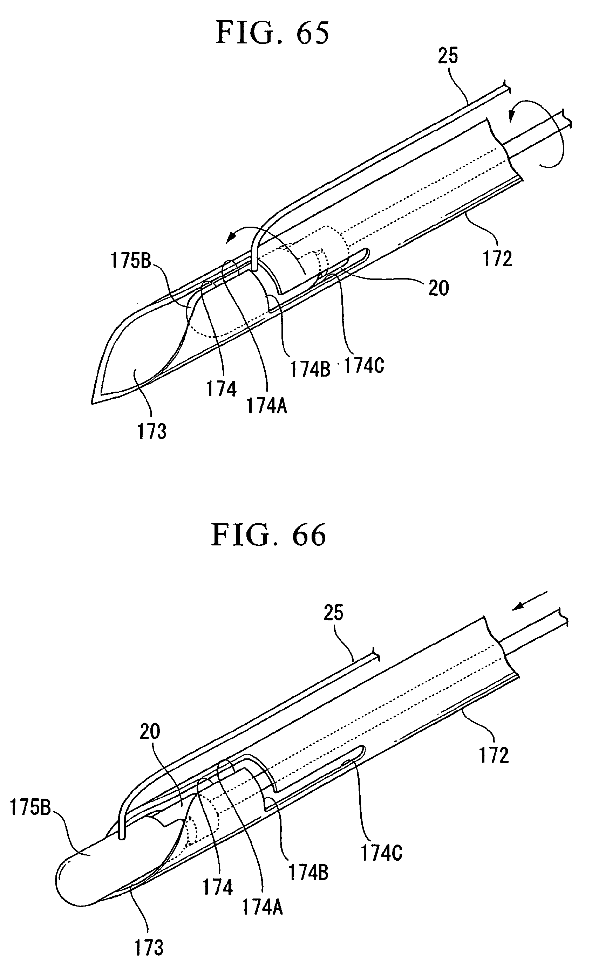

To push out the second anchor 175B, the pusher 20 is rotated. Since the pusher 20 and the second anchor 175B are connected in the rotation direction by the engagement portions 177, the second anchor 175B rotates with the pusher 20 as shown in FIG. 65, and the suture thread 25 thereof moves through the second portion 174B of the side hole 174 to the first portion 174A. In this state, by further moving forward the pusher 20, the suture thread 25 moves along the first portion 174A as shown in FIG 66, and the second anchor 175B is pushed out from the needle 172. Since the engagement portion between the pusher 20 and the second anchor 175B does not engage in the pullback direction of the pusher 20, the second anchor 175B separates from the pusher to be released from the needle 172.

In this example, the crank-shaped side hole 174 is provided as a push-out control portion, the first anchor 175A is disposed at the distal end side, the second anchor 175B is disposed at the base end side, and by providing the pusher 20 that engages with the second anchor 175 in the rotation direction, it is possible to reliably push out only the first anchor 175A.

### [Twelfth example]

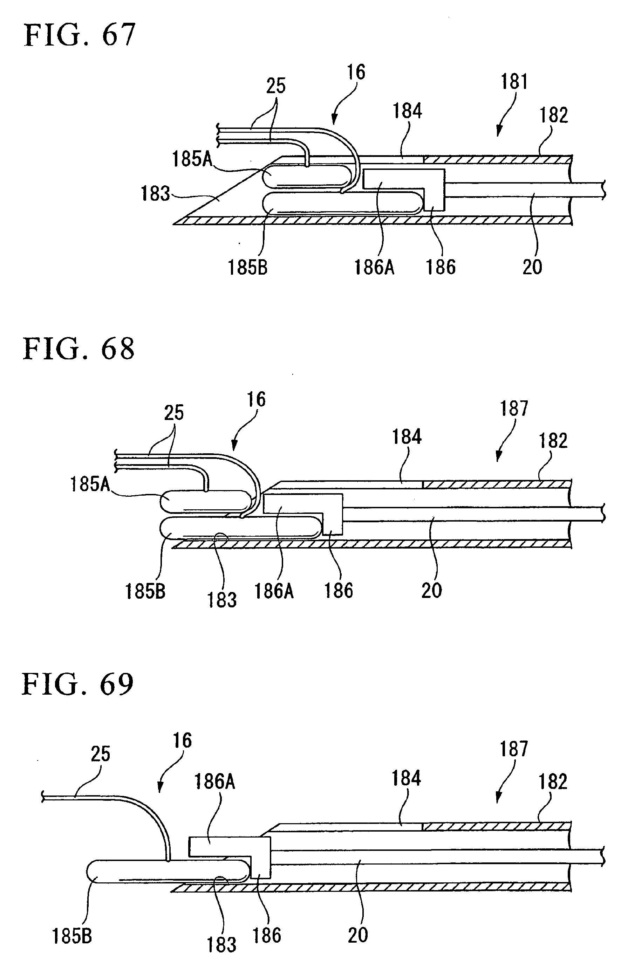

As shown in FIG 67, an opening portion 183 is obliquely formed at the sharp tip of the distal end of a needle 182 of a suture tool 181, and a side hole 184 extends in the lengthwise direction from the base end side of the opening portion 183. In the needle 182, a first anchor 185A and a second anchor 185B that is longer than the first anchor 185A of the suture tool 16 are disposed in a manner arranged in the radial direction of the needle 182. The first anchor 185A is disposed at the base end side of the opening portion 183 of the needle 182, that is, on the side of the side hole 184. A distal end portion 186 (push-out control portion) of the pusher 20 has a step, and an end portion 186A on the side of the side hole 184 projects toward the distal end. This distal end portion 186 of the pusher 20 makes contact with both anchors 185A, 185B.

To push out the first anchor 185A, the pusher 20 is moved forward. The two anchors 185A, 185B are pushed simultaneously by the distal end portion 186 of the pusher 20, but the length of the first anchor 185A is short and is in the opening direction of the opening portion 183. Accordingly, as shown in FIG 68, the entire length of the first anchor 185A projects to the outside of the needle 182 before the second anchor 185B. As a result, the first anchor 185A is pushed out first. At this time, the second anchor 185B is hardly exposed from the needle 182, or completely unexposed. Moreover, since the second anchor 185B is sandwiched between the needle 182 and the end portion 186A of the pusher 20, it does not fall out of the needle 182. To push out the second anchor 185B, the pusher 20 is moved further forward. As shown in FIG 69, by projecting almost the entire length of the second anchor 185B from the needle 182, it is released from the suture tool 181.

In this example, since the distal end portion 186 of the pusher 20 is provided as a push-out control portion, it is possible to push out the two anchors 185A, 185B with different lengths in sequence. Since the first anchor 185A is shorter than the second anchor 185B and is disposed so as to protrude first from the opening portion 183, it is possible to reliably push out the first anchor 185A before the second anchor 185B.

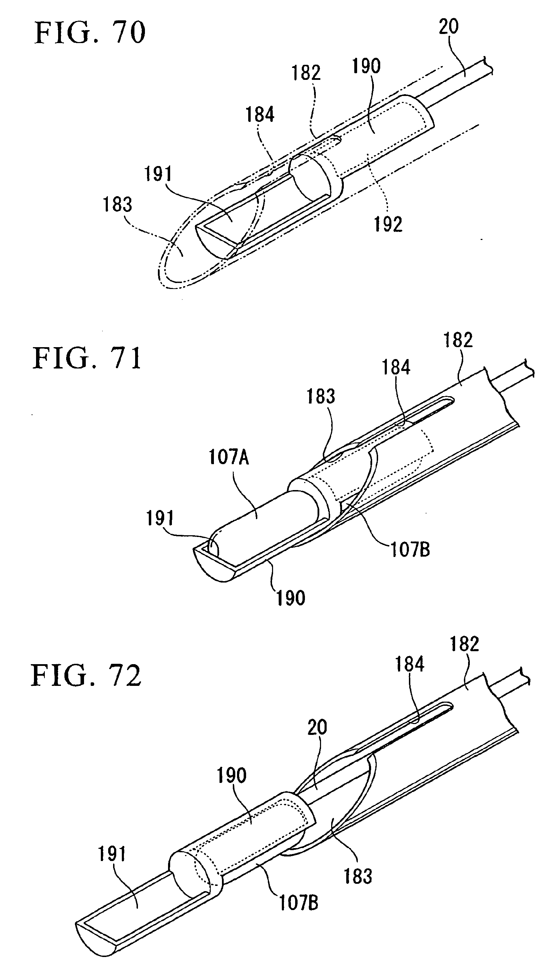

Here, modification examples are shown in FIG 70 to FIG 72. A distal end portion 190 (push-out control portion) of the pusher 20 has an outer shape in which a cylindrical distal end side and base end side are each cut into a half-circle shape at one location. At the distal end side, the opening direction side of the opening portion 183 that is sloped is cut away, and a recessed portion 191 that houses one elongated anchor of the suture tool 16 is formed. A recessed portion 192 at the base end side is formed by cutting away the side opposite the recessed portion 191 of the distal end side. This recessed portion 192 can also house one anchor.

As shown in FIG 71, to push out a first anchor 107A, the pusher 20 is moved forward until approximately the entire length of the first anchor 107A projects from the opening portion 183. Thereby, the first anchor 107A that is disposed in the opening direction is released from the needle 182. At this time, since the second anchor 107B is between the recessed portion 192 of the pusher 20 and the needle 182, it is not released from the needle 182. To push out the second anchor 107B, as shown in FIG. 72, the pusher 20 is moved forward until approximately the entire length of the second anchor 107B projects from the needle 182.

By providing the distal end portion 190 of the pusher 20 as a push-out control portion, it is possible to differentiate the timing at which the two anchors 107A, 107B project to be capable of separating, and so it is possible to reliably push out the anchors 107A, 107B one at a time.

### [Thirteenth example]

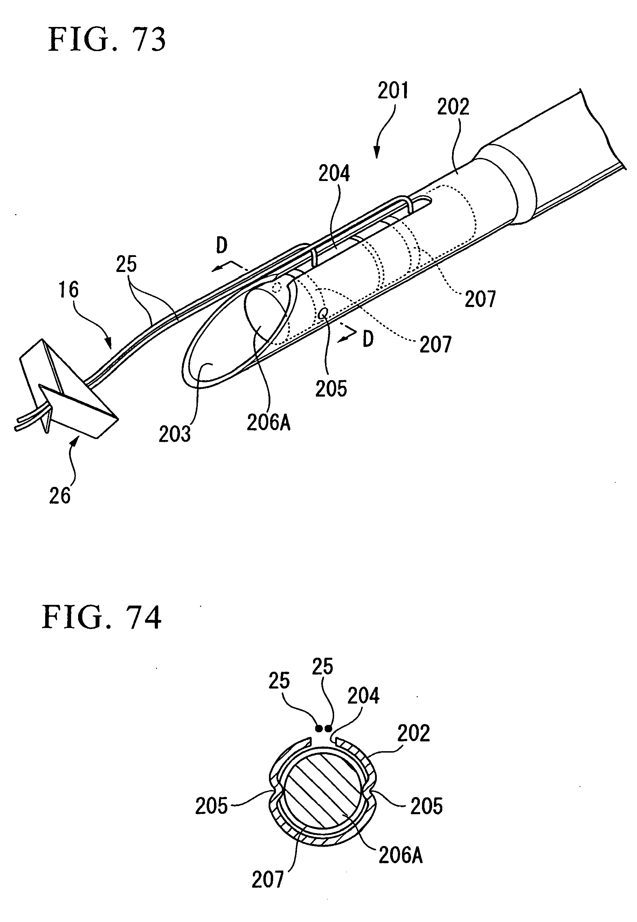

As shown in FIG 73 to FIG 75, an opening portion 203 is obliquely formed at the sharp tip of the distal end of a needle 202 of a suture tool 201, and a side hole 204 extends in the lengthwise direction from the base end side of the opening portion 203. Two projections 205 (push-out control portions) are formed in the needle 202 facing the inner side. It is preferable that the needle 202 be manufactured from a material that has resilience. Note that by providing the side hole 204, the resilience is enhanced.

A first anchor 206A and a second anchor 206B of the suture tool 16 have an elongated shape, and a groove 207 is formed in an annular shape on the outer circumference of each. The grooves 207 are of a size to be capable of engaging with the projections 205 of the needle 202. In the initial state, the projections 205 of the needle 202 are engaged with the groove 207 of the first anchor 206A on the distal end side.

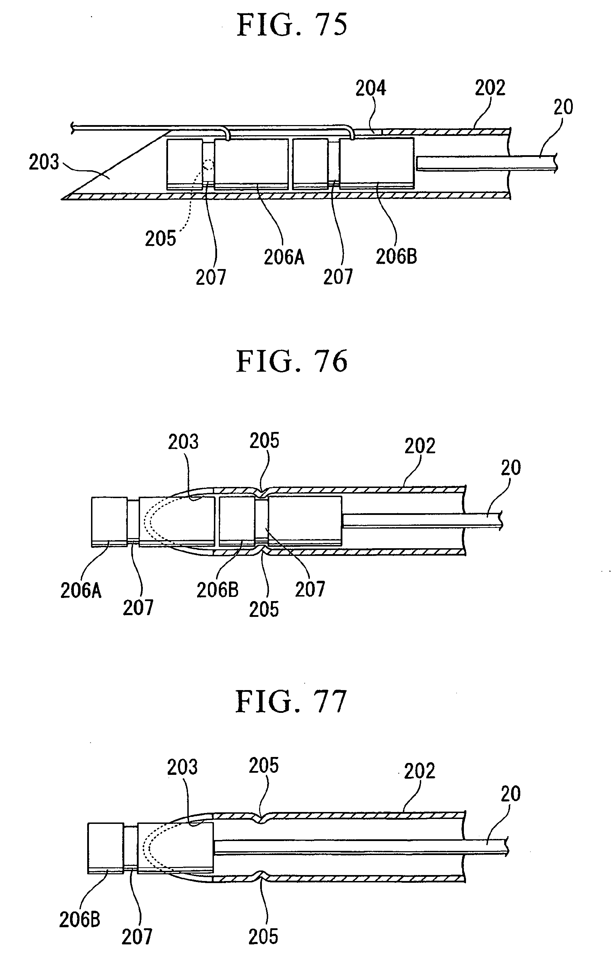

To push out the first anchor 206A, the pusher 20 is moved forward. The first anchor 206A proceeds past the protrusions 205, and so the engagement with the protrusions 205 is released. The amount of force required for releasing the engagement is the magnitude that can be input from the hand side. As shown in FIG. 76, when the pusher 20 is moved forward as is, the first anchor 206A is pushed out from the opening portion 203 of the distal end. The second anchor 206B stops when the groove thereof engaged with the projections 205. For this reason, only the first anchor 206A is pushed out. To push out the second anchor 206B, the pusher 20 is moved further forward. As shown in FIG. 77, the second anchor 206B is thereby pushed out similarly to the case of the first anchor 206A.

According to this example, by providing the projections 205 on the side of the needle 202 as a push-out control portion and making the groove 207 of the anchors 206A, 206B engage, it is possible to reliably push out the first anchor 206A only. Note that in order to change the operational feeling when pushing out the first anchor 206A and when pushing out the second anchor 206B, a difference in the force amount may be provided by pushing out the anchors without providing the groove 207 in the first anchor 206A.

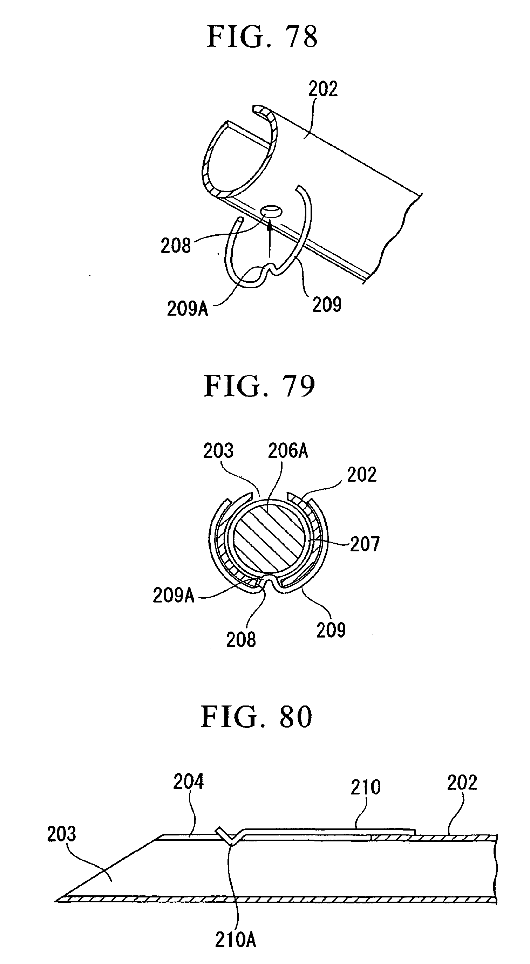

Here, embodiments are shown in FIG. 78 to FIG 80.

As shown in FIG. 78 to FIG 79, it is acceptable to form a hole 208 in the needle 202 instead of the projections and mount a C-shaped ring 209 (push-out control portion) with a projection portion 209A formed in the center thereof at the hole 208. When the projection portion 209A of the ring 209 is mounted on the needle 202, it projects into the needle 202 via the hole 208. This projection portion 209A accomplishes a similar function as the projections 205.

In the needle 202 shown in FIG. 80, a wire spring 210 (push-out control portion) is provided to the base end side than the side hole 204. The wire spring 210 extends toward the distal end, and an engagement portion 210A is formed that is folded back to face the inside of the side hole 204. This engagement portion 210A accomplishes a similar function as the projections 205. This push-out control portion is not limited to a line shape provided it is elongated, and may be a plate shape or ribbon shape.

### [Fourteenth example]

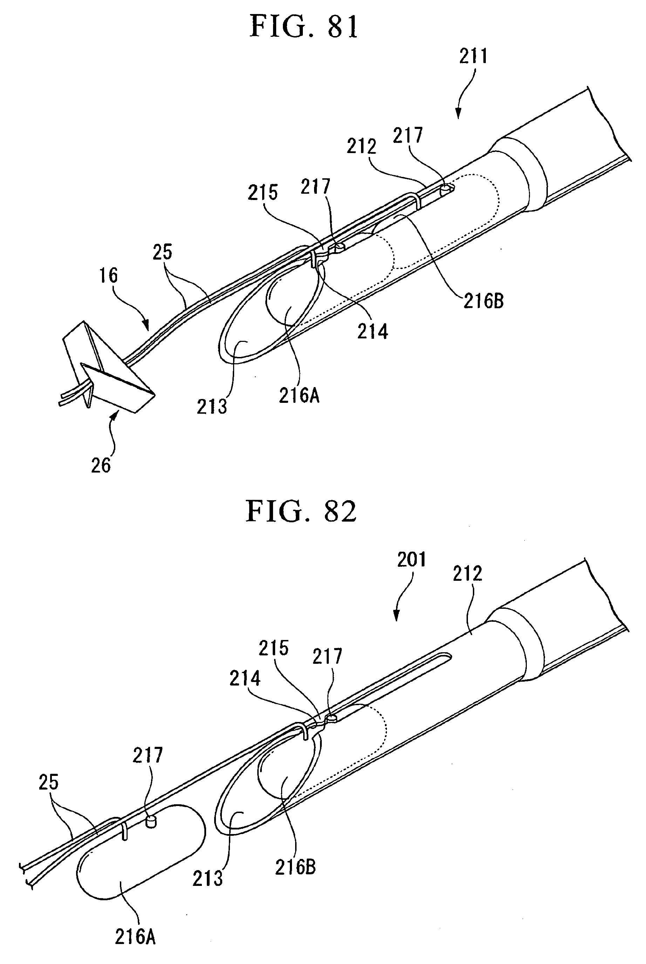

As shown in FIG 81, an opening portion 213 is obliquely formed at the sharp tip of the distal end of a needle 212 of a suture tool 211, and a side hole 214 is formed from the base end side of the opening portion 213 along the lengthwise direction. In the side hole 214, a narrowed portion 215 is formed in which the opening length becomes smaller at the distal end side. The narrowed portion 215 is constituted by a pair of projected portions 217 that form a smooth curve. Two anchors 216A, 216B of the suture tool 16 that are housed in the needle are each provided with a projection 217 on the outer circumference portion of the elongated shape. The projection 217 is provided at a position that is shifted to the base end side from a location where the suture thread 25 is drawn out. The diameter of the projection 217 is larger than the opening length of the narrowed portion 215 when an external force is not acting on the narrowed portion 215 of the needle 212. Accordingly, the projection 217 is caught on the narrowed portion 215, thereby preventing drop off of the anchors 216A, and 216B.

To push out the first anchor 216A, the pusher 20 is moved forward. Thereby, the projection 217 of the first anchor 216A moves forward by spreading out the narrowed portion 215, and is pushed out from the opening portion 213 of the distal end. As shown in FIG. 82, after the projection 217 has passed, the narrowed portion 215 reverts to its original shape, and so the projection 217 of the second anchor 216B is caught and engaged. In this way, by providing the narrowed portion 215 as a push-out control portion, it is possible to provide a difference in the force amount, and it is possible to reliably push out only the first anchor 216A. To push out the second anchor, a force is newly added to spread open the narrowed portion 215. Note that instead of spreading open the narrowed portion 215 with the projection 217, it is possible to spread open the narrowed portion 215 with the suture thread 25.

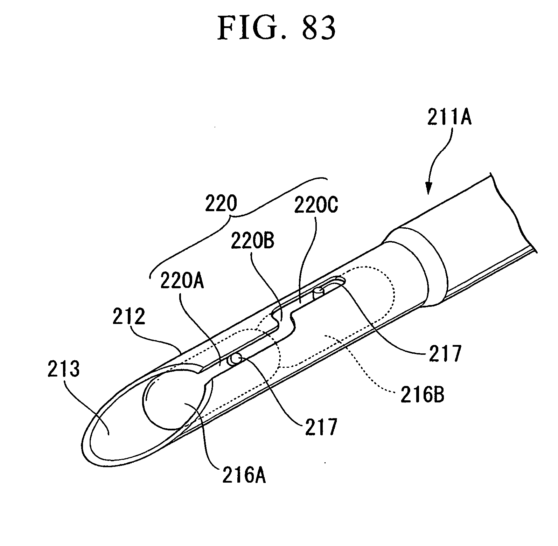

FIG. 83 shows a modified example. The side hole 220 has a cam configuration having a first portion 220A of the distal end side, passes through a connecting portion 220B that smoothly curves, and links to a second portion 220C on the base end side. In the initial state, the suture thread 25 and the projection 217 of the first anchor 216A are passed through the first portion 220A, and the suture thread 25 and the projection 217 of the second anchor 216B are passed through the second portion 220C. The length of the portions 220A to 220C of the side hole 220 is set so as to be capable of pushing out the first anchor 216A while the projection 217 of the second anchor 216B is in the second portion 220C.

The first anchor 216A is pushed out if the pusher 20 is moved forward. Since the projection 217 of the second anchor 216 remains in the second portion 220C of the side hole 220, the second anchor 216B is not pushed out simultaneously. To push out the second anchor 216, the projection 217 thereof must be passed through the curved connecting portion 220B of the side hole 220, and so the work becomes heavy from the standpoint of the operator. When the connecting portion 220B is passed, the second anchor 216B once again moves forward with a small force, and is pushed out from the needle 212. In this way, by providing the side hole 220 as a push-out control portion, a difference in the force amount is provided when pushing out the two anchors 216A, 216B, and it is possible to reliably control the push out of the two anchors 216A, 216B.

### [Fifteenth example]

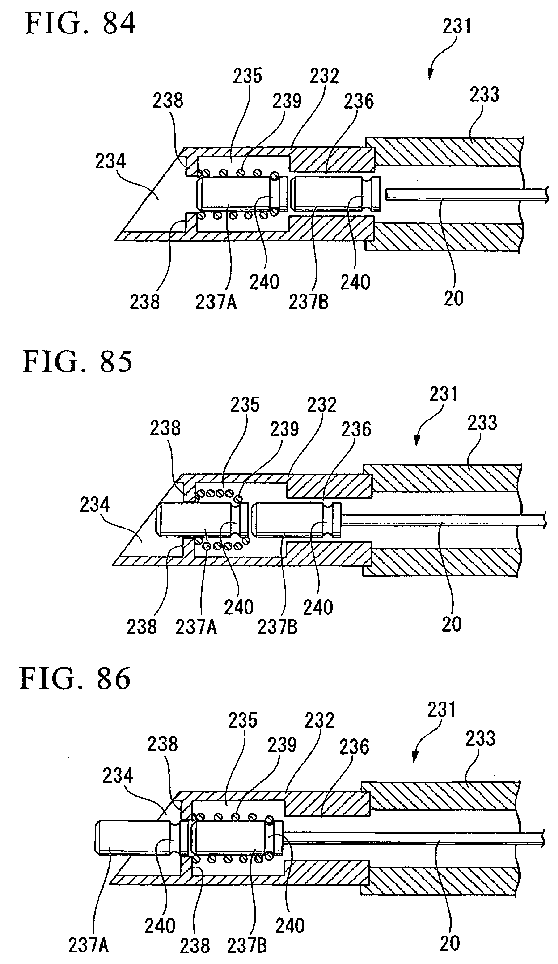

As shown in FIG. 84, a needle 232 of a suture instrument 231 is fixed at the distal end of a sheath 233. An opening portion 234 is obliquely formed at the sharp tip of the distal end of the needle 232. The interior of the needle 232 contains, at the distal end side, a first housing portion 235 with a large diameter and, at the base end side, a second housing portion 236 with a small diameter. In the initial state, an elongated first anchor 237A of the suture tool 16 is housed in the first housing portion 235, and an elongated second anchor 237B of the suture tool 16 is housed in the second housing portion 236. The first housing portion 235 continues into the second housing portion 236, with the distal end side being partitioned by an annular wall portion 238 that projects the inner wall of the needle 232. Moreover, a coil-shaped spring 239 is housed in the first housing portion 235. The outer diameter of the distal end side of the spring 239, in the state of an external force not acting thereon, is larger than the outer diameter of the first anchor 237A and the inner diameter of the wall portion 238. The base end portion of the spring 239 is reduced in diameter, and engages with a groove 240 that is cut in the outer circumference of the first anchor 237. Thereby, the movement of the first anchor 237A is regulated by the distal end portion of the spring 239 abutting the wall portion 238, and thus the first anchor 237A remains in the first housing portion 235. In the two anchors 237A, 237B, the groove 240 is formed in the base end of each, and the outer circumference of the distal end portion has a reduced diameter by beveling.

When the pusher 20 is moved forward, the first anchor 237A moves toward the opening portion 234 of the distal end, and the distal end portion of the spring 239 is thrust against the wall portion 238. When the pusher 20 is pushed further forward, since the distal end portion of the spring 239 abuts the wall portion 238 and does not move while the base end side of the spring 239 advances with the first anchor 237A, the spring 239 is compressed and increases in diameter. As a result, as shown in FIG 85, the base end portion of the spring 239 expands in diameter to come off the groove 240 of the first anchor 237A, and so the first anchor 237A is pushed out from the distal end of the needle 232. As a result of the first anchor 237A being pushed out, the second anchor 237B advances into the first housing portion 235. At this time, due to the beveled distal end of the second anchor 237B, the base end portion of the spring 239 expands in diameter. When the second anchor 237B advances as is, the base end portion of the spring 239 moves relatively along the outer circumference of the second anchor 237B, and as shown in FIG 86, engages with the groove 240 of the second anchor 237B. Thereby, since the movement of the second anchor 237B is restricted, the two anchors 237A, 237B are not pushed out simultaneously.

This example has the spring 239 as a push-out control portion and controls the movement of the anchors 237A, 237B by utilizing the expansion in the radial direction of the spring 239 when compressed. Therefore, it is possible to reliably push out the anchors 237A, 237B one at a time. The spring 239 reverts to its original shape after the anchors 237A, 237B have been pushed out, and so can be repeatedly used.

### [Sixteenth example]

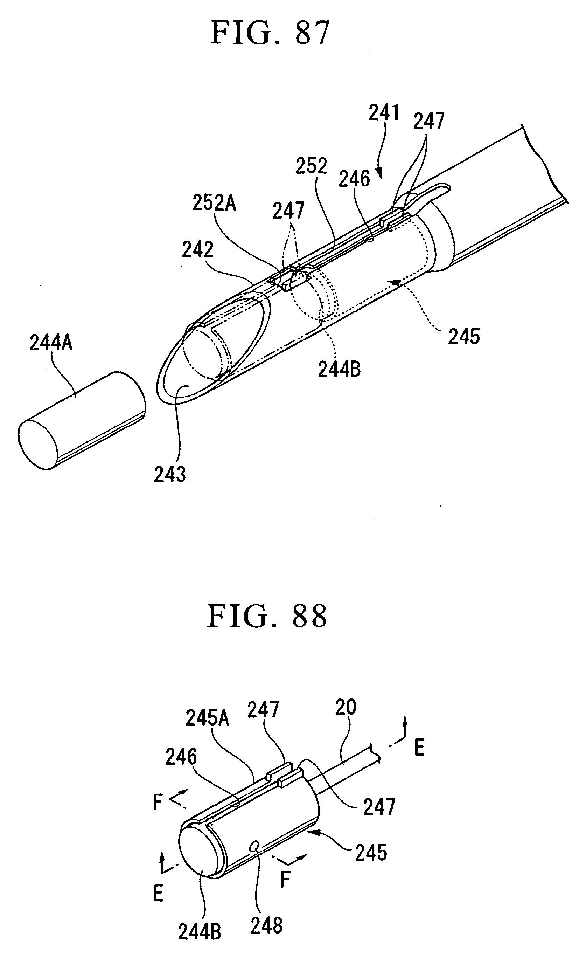

As shown in FIG. 87 and FIG 88, an opening portion 243 is obliquely formed at the sharp tip of the distal end of a needle 242 of a suture tool 241. An elongated first anchor 244A and a second anchor 244B of the suture tool 16, with the second anchor 244B having a smaller diameter than the first anchor 244A, are housed in the needle 242 in that order from the distal end side. The second anchor 244B is housed in a housing portion 245 (intermediate member) that is provided at the distal end portion of the pusher 20.

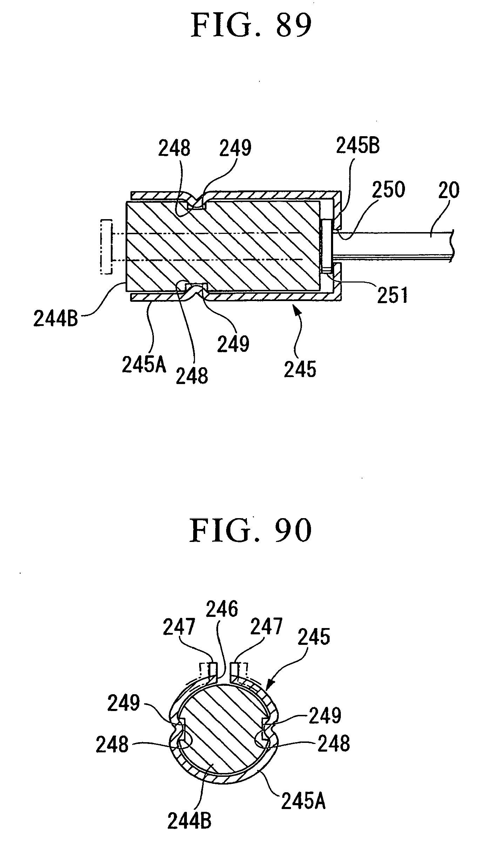

The housing portion 245 is manufactured from a material that has resilience and is formed in a cylindrical shape with a bottom. A slit 246 is formed in a cylindrical portion 245A of the housing portion 245 in the lengthwise direction thereof. At the base end portion of the cylindrical portion 245A, a pair of engagement projections 247 is provided sandwiching the slit 246, with each engagement projection 247 extending to the outside in the radial direction. Moreover, on the inner side of the cylindrical portion 245A of the housing portion 245, two projections 248 are formed facing inward. These projections 248 engage with the recessed portions 249 of the second anchor 244B. As shown in FIG 89, a hole 250 is formed at a bottom portion 245B of the housing portion 245. The hole 250 passes the pusher 20 to be able to freely move forward or backward, while a portion 251 with a wide diameter at the distal end of the pusher 20 is of a size that cannot be inserted.

As shown in FIG. 87, a side hole 252 is formed in the needle 242 along the lengthwise direction thereof. A widened portion 252A is formed at the distal end of the side hole 252. The width along the circumferential direction of the widened portion 252A is greater than the distance between the pair of engagement projections 247 of the housing portion 245. The width of the remainder of the side hole 252 excluding the widened portion 252A is smaller than the length between the pair of engagement projections 247 in the natural state. As shown in FIG. 90, when the engagement projections 247 are made to approach each other to the extent of making contact, they can be passed through the side hole 252. At this time, the projections 248 of the housing portion 245 engaged with the recessed portions 249 of the second anchor 244B. In contrast, at the widened portion 252A of the side hole 252, the pair of engagement projections 247 is restored to the original state so as to open as shown by the dashed lines. At this time, the projections 248 of the housing portion 245 move to the outside in the radial direction, and so their engagement with the recessed portions 249 of the second anchor 244B is released.

To push out the first anchor 244A, the pusher 20 is moved forward. The first anchor 244A is pushed out from the needle 242 via the second anchor 244B, which is pushed by the pusher 20, and the housing portion 245. Since the engagement projections 247 of the housing portion 245 are biased so as to approach each other by the side hole 252, the projections 248 maintain the state of engagement with the recessed portions 249 of the second anchor 244B, and so the housing portion 245 advances with the second anchor 244B. For this reason, even when the first anchor 244A is pushed out from the needle 242, the second anchor 244B is not simultaneously pushed out.

When the pusher 20 is further moved forward after pushing out the first anchor 244A, the engagement projections 247 enter the widened portion 252A of the side hole 252. Since the force that had biased the engagement projections 247 toward each other is removed, the cylindrical portion 245A of the housing portion 245 opens, and the engagement between the engagement projections 247 and the recessed portions 249 of the second anchor 244B is released. In this state, when the pusher 20 is moved further forward, the second anchor 244B is pushed by the portion 251 at the distal end of the pusher 20. In contrast, the housing portion 245, which is not engaged with either of the pusher 20 and the second anchor 244B, does not move since the side hole 252 does not extend further to the distal end side. Accordingly, the second anchor 244B is pushed out from the needle 242 accompanying the advance of the pusher 20.

In this example, by adjusting the engagement state between the housing portion 245 and the second anchor 244B, and the pusher 20, it is possible to ensure that the second anchor 244B does not come out from the housing portion 245 when pushing out the first anchor 244A. Accordingly, it is possible to control the pushing out of the two anchors 244A, 244B.

Also, after the first anchor 244A has been pushed out, it is possible to draw back the second anchor 244B by pulling the pusher 20. For this reason, in the case of the puncturing performance of the needle 242 being affected by the second anchor 244B being at the distal end portion of the needle 242, it is possible to improve the puncturing performance.

Here, a modification example of the present example shall be shown.

The second anchor 244B may be made from a magnetic body, and the housing portion 245 may be made from a permanent magnet. In this case, the pusher 20 is made from a non-magnetic body. The second anchor 244B and the housing portion 245 are thus engaged by the attraction due to the magnetism, and when a force is applied that overcomes this magnetism, the second anchor 244B can be pushed out. Alternatively, the housing portion 245 may be made from a magnetic body, and the second anchor 244B may be a permanent magnet.

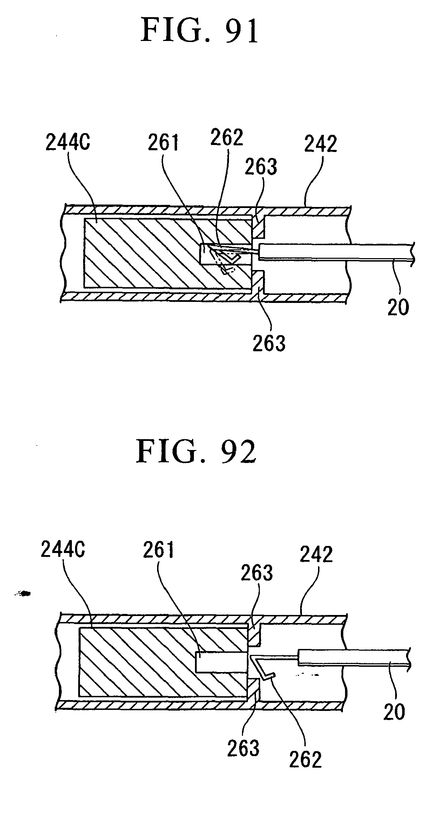

As shown in FIG 91, a recessed portion 261 that extends in the lengthwise direction may be formed in the center of the base end portion of an elongated second anchor 244C. A hook 262 at the distal end of the pusher 20 is pushed into this recessed portion 261. When an external force is not applied to this hook 262, it is wider than the diameter of the hole. In the needle 242 is provided a wall portion 263 that is capable of abutting the base end portion of the second anchor 244C and allows the pusher 20 to move forward and backward.

To push out the first anchor 244A, since the second anchor 244C is engaged by the hook 262, the second anchor 244C moves forward with the pusher 20. After pushing out the first anchor 244A, the pusher 20 is made to retreat. The second anchor 244C that is engaged with the pusher 20 is drawn back. When the second anchor 244C abuts the wall portion 263, it can move no longer back, but the pusher 20 can be moved further back. As shown in FIG 92, the hook 262 of the pusher 20 is pulled out from the recessed portion 261 of the second anchor 244C, and thus the engagement of both is released. When the hook 262 reverts to its original shape, it becomes bigger than the recessed portion 261. Accordingly, when the pusher 20 is moved forward once again, the second anchor 244C can be pushed out with the hook 262.

### [Seventeenth example]

In this example, the case of using the endoscope 1 and the suture instrument 11 the same as those in the first example are described as another suturing method, but a suture instrument of another example may also be used.

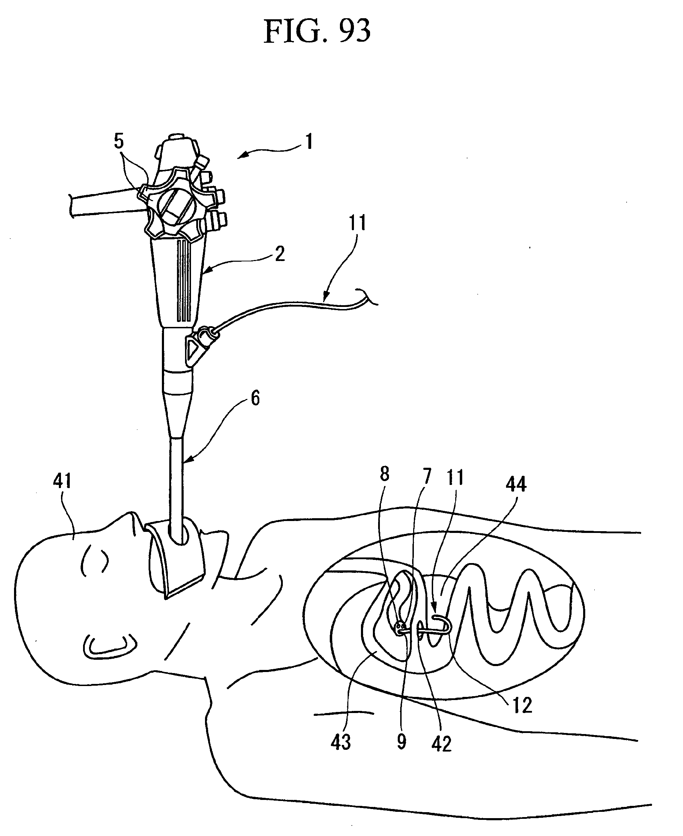

The suture method of this example shall be described. As shown in FIG. 6, the endoscope insertion part 6 is inserted until the vicinity of the perforation 42, and performs observation of the perforation 42 from within the stomach 43. Next, as shown in FIG. 7, the endoscope insertion part 6 is fed from the perforation 42 into the abdominal cavity 44, and observation of the perimeter of the perforation 42 is made from the abdominal cavity 44 side with an observation device (first observation device) 7 of the endoscope insertion part 6. After confirmation that other tissue is not in the area of the perforation 42 (the position through with the needle 14 passes, the puncture position, or the position where the anchor 27 is placed), the endoscope insertion part 6 is drawn back into the stomach 43. Next, the suture instrument 11 that is passed through the channel 9 is projected out. As shown in FIG. 93, the distal end portion of the suture instrument 11 is fed from the perforation 42 into the abdominal cavity 44. Then, the distal end portion of the suture instrument 11 is curved and made to face the exterior side of the stomach 43 in the abdominal cavity 44 and the perimeter of the perforation 42.

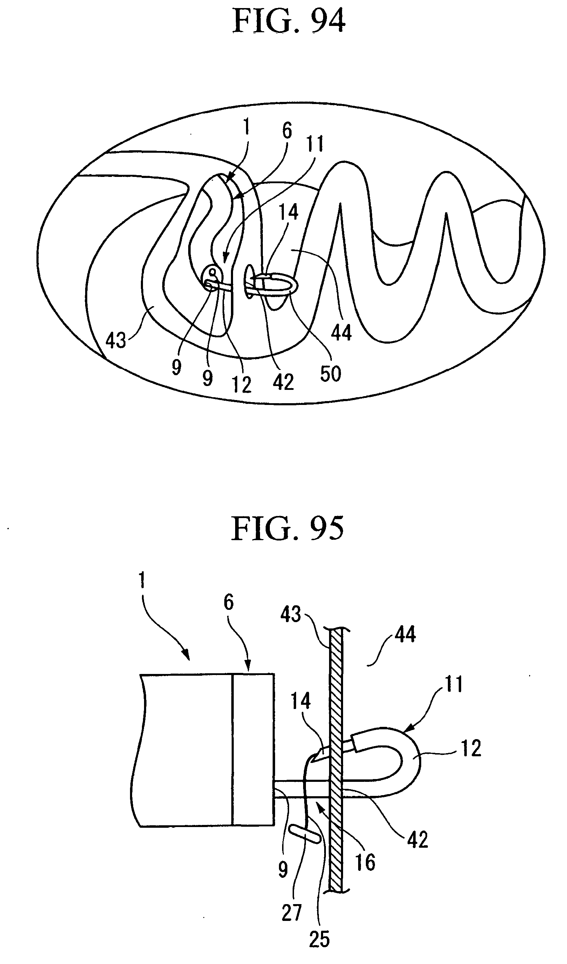

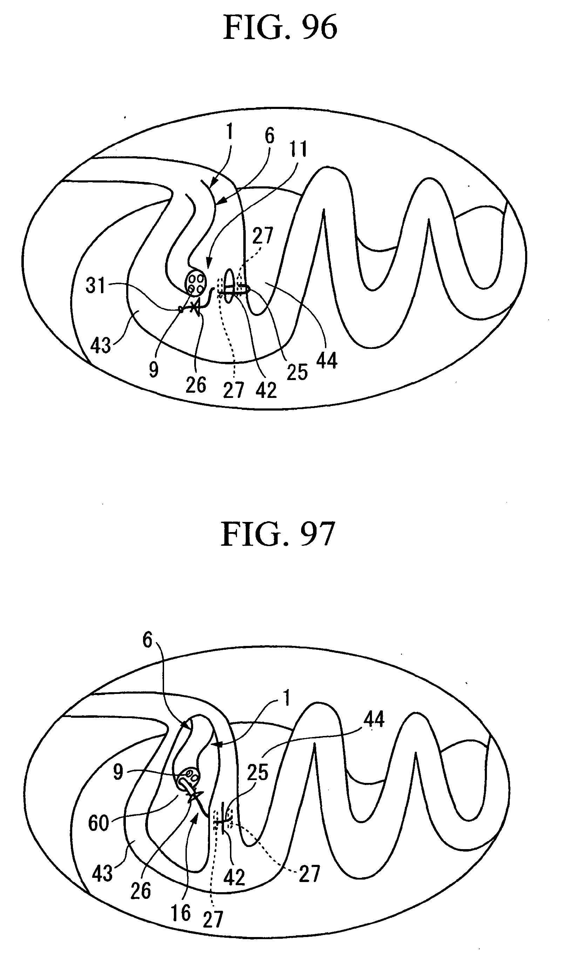

As shown in FIG 94, the needle 14 of the suture instrument 11 is projected from the outer sheath 12, and the needle 14 passes through the tissue on the perimeter of the perforation 42 from the side of the abdominal cavity 44 into the stomach 43. When projecting the needle 14 from the outer sheath 12, it is preferable that the stopper 26 enters the stomach 43. As shown in FIG 95, when the needle 14 has passed through the tissue, a first anchor 27 is pushed out to the inside of the stomach 43 and made to remain there. As shown in FIG 96, two anchors 27 are placed in the stomach 43 so as to sandwich the perforation 42, and then the suture instrument 11 is returned to inside of the stomach 43 to be housed in the channel 9. Then, as shown in FIG 97, the forceps 60 are passed through the channel 9, and the tissue is fastened with the suture tool 16 using the forceps 60, whereby the perforation 42 is sutured. This fastening method is the same as that of the second example.

In this example the interior side and exterior side of the stomach 43 are observed in sequence by the observation device 7 of the endoscope 1 to confirm that there is no other tissue around the perforation, the endoscope 1 is then returned to the inside of the stomach 43, and the needle 14 is passed through tissue from the exterior side of the stomach 43. Accordingly, the inclusion of other tissue when performing suturing using the endoscope 1 can be readily prevented.

### [Eighteenth example]

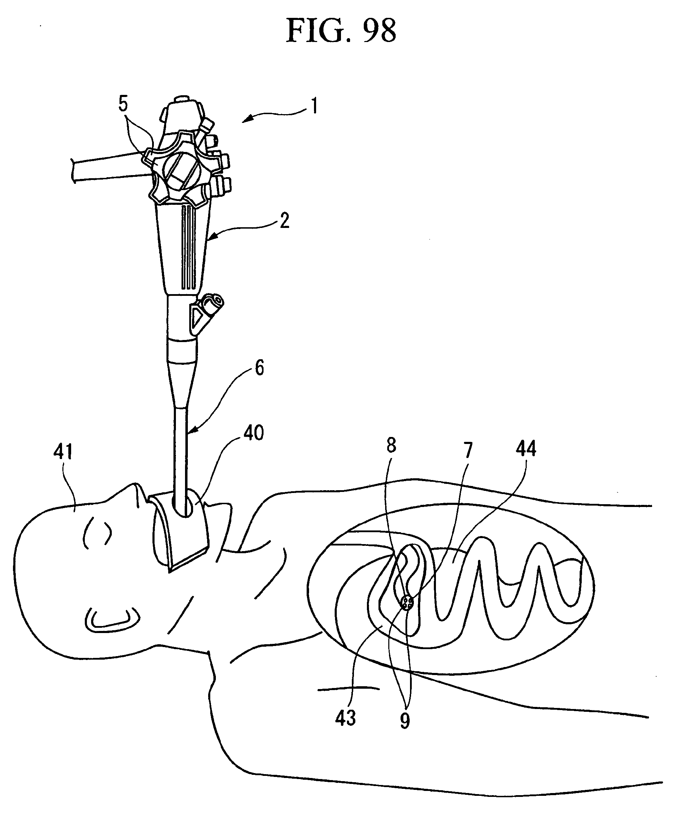

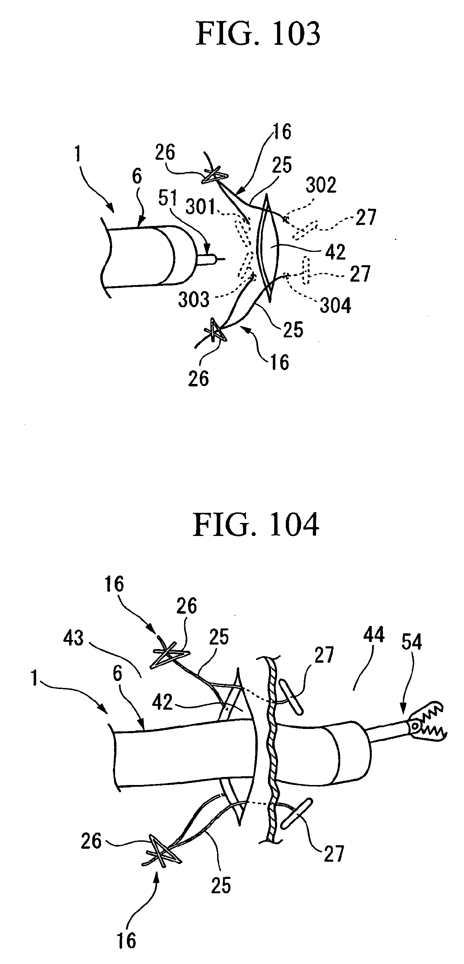

In this example, the case of using the endoscope 1 and the suture instrument 11 the same as those in the first example are described as another suturing method, referring mainly to FIG 98 to FIG 114, but a suture instrument of another example may also be used. Note that FIG 98 to FIG 114 are schematic views showing procedures, and a stomach is shown as an example of a hollow organ.

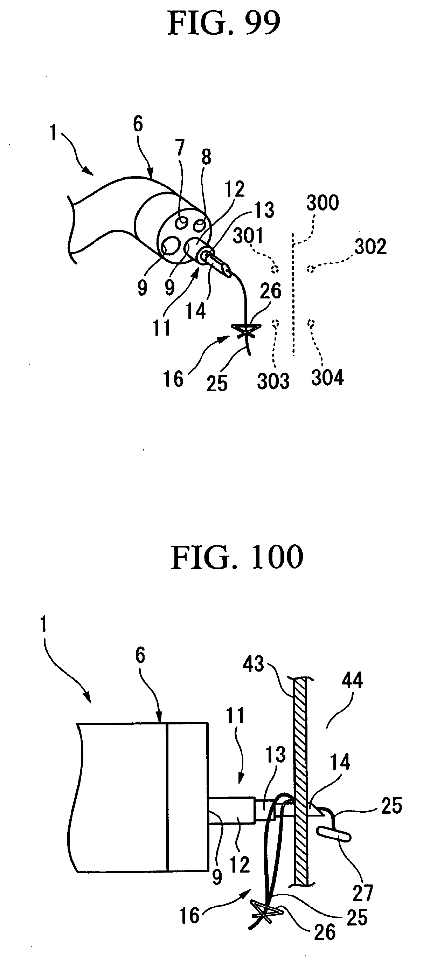

As shown in FIG. 98, the endoscope insertion part 6 is inserted from the mouth (including a natural opening of a living body, anus, nose, ear and the like) of the patient 41 prepared with a mouthpiece 40, and the distal end of the endoscope insertion part 6 is made to bend with the angle knob 5. An intended incision line 300 shown by virtual lines in FIG. 99 is confirmed with the observation device 7 from the interior side of the stomach 43 (the interior side of the hollow organ), and the intended incision line 300 where the incision is to be made afterward is set at this intended incision line 300. When doing so, the intended incision line 300 may be marked with a high-frequency knife. As shown in FIG. 99, the suture instrument 11 is projected out, the needle 14 is pushed out from the outer sheath 12, and the stopper 26 is lowered into the stomach 43. The inner sheath 13 of the suture instrument 11 is moved forward, and furthermore puncture positions 301, 302, 303, 304 are determined as suture positions when suturing with the suture instrument 11 based on the intended incision line 300. The puncture position 301 and the puncture position 302 are symmetrical positions about the intended incision line 300, and a straight line through the two puncture positions 301, 302 approximately perpendicularly intersects the intended incision line 300. The same is true for the puncture positions 303, 304. At that time, marking of the intended incision line 300 may be performed with a high-frequency knife or the like.

Once the puncture positions 301, 302, 303, 304 have been determined, the needle 14 is thrust at the initial puncture position 301. As shown in FIG. 100, when the needle 14 has passed through the wall portion of the stomach 43, the handle 19 on the hand side shown in FIG. 1 is pushed in to move the pusher 20 forward. The pusher 20 pushes out the first anchor 27 from the distal end of the needle 14 into the exterior side of the stomach 43 (also referred to as the body cavity side or abdominal side of the hollow organ). Once the first anchor 27 has been pushed out, the handle 19 is stopped, and the inner sheath 13 is drawn in. The needle 14 is then drawn out of the wall portion, so that only the suture thread 25 passes through the wall portion, and the first anchor 27 remains on the side of the abdominal cavity 44.

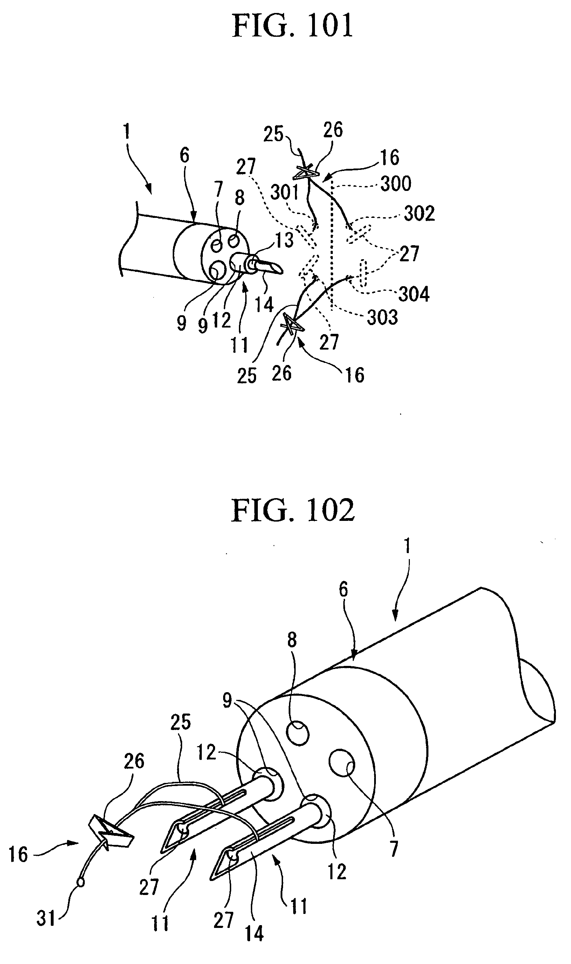

Next, the needle 14 is thrust at the puncture position 302 which is a symmetrical position to sandwich the intended incision line 300 shown in FIG. 99. The second anchor 27 is similarly pushed out to the abdominal cavity 44. As shown in FIG. 8, a separate suture tool 16 is similarly mounted at the puncture position 303 and the puncture position 304. When mounting the two suture tools 16, it is acceptable to pull the suture instrument 11 out from the endoscope 1 each time one suture tool 16 is mounted and then pass the suture instrument 11 that houses the new suture tool 16 through the endoscope 1. Also, as shown in FIG. 102, it is also acceptable to pass the suture instrument 11 in advance in each of two channels 9, and so mount the suture tools 16 in sequence.

When the suture tools 16 are mounted, the intended incision line 300 is cut. As shown in FIG. 102, a treatment tool for cutting, for example a needle-shaped knife 51 that is a high-frequency incision tool, is passed through the channel 9 of the endoscope insertion part 6. A high-frequency is impressed on the distal end portion of the needle-shaped knife 51, and when this needle-shaped knife 51 is moved along the intended incision line 300, the wall portion is incised and the perforation 42 is formed. As shown in FIG. 11, the endoscope insertion part 6 is fed to an abdominal cavity 53 via the perforation 42. A treatment tool such as forceps 54 is passed through the channel 9 to perform a medical procedure in the abdominal cavity 53.

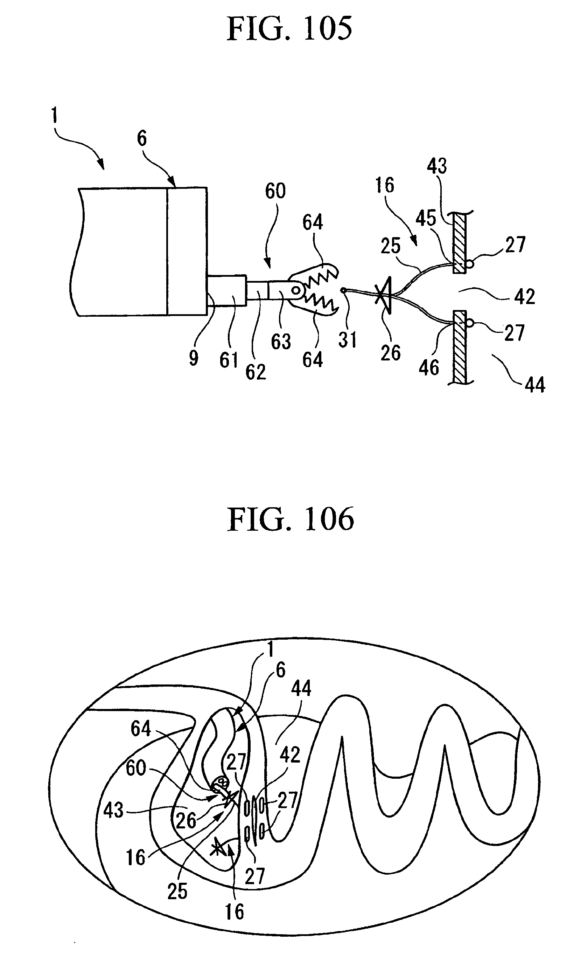

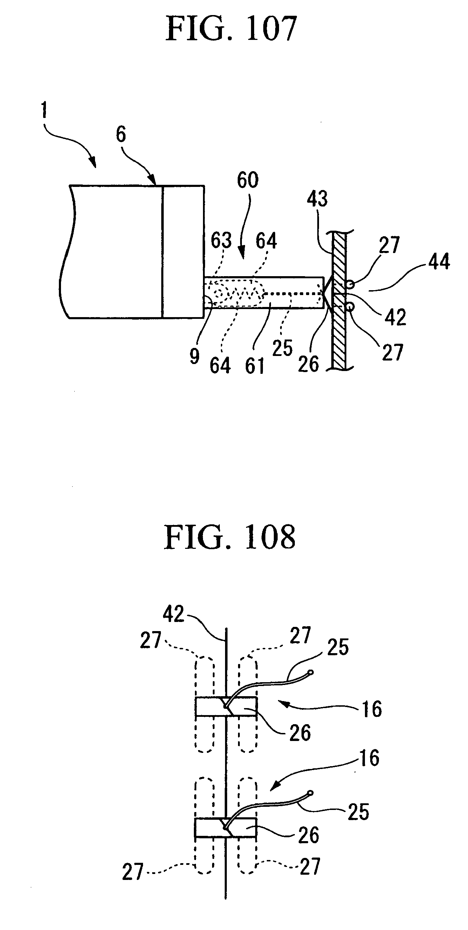

After completing the medical procedure in the abdominal cavity 53, the endoscope insertion part 6 is drawn back into the stomach 43, and the perforation 42 is sutured by fastening the two suture tools 16. At this time, for example, forceps 60 such as those shown in FIG. 105 are used. The forceps 60 have an outer sheath 61 that has a larger diameter than the anchor 27, and the inner sheath 62 is passed through the inside of a flexible outer sheath 61 so as to be able to freely move forward or backward. At the distal end of the inner sheath 62, a supporting member 63 is provided, and a pair of grip segments 64 is supported on the supporting member 63 so as to freely open or close. As shown in FIG. 106, after the knot 31 of the suture thread 25 of the suture tool 16 is gripped by the grip segments 64, the outer sheath 61 is moved forward and the distal end of the outer sheath 61 presses against the stopper 26. As shown in FIG 107, when the outer sheath 61 moves further forward, the stopper 26 is pushed into the wall portion of the stomach 43. Since the stopper 26 is constructed to be able to move in this direction, the stopper 26 moves toward the wall portion 45. As a result, the distance between the stopper 26 and the anchor 27 decreases. Thereby, the tissue around the perforation 42 is pulled together, and the perforation 42 is sutured by the suture thread 25. When the perforation 42 has been sutured by the suture tool 16, after the outer sheath 61 is retracted, the grip segments 64 are opened to release the suture thread 25. The end portion of the stopper 26 is capable of moving in the direction of fastening the tissue with the suture thread 25, but by working so as to tighten the suture thread 25 in the direction of loosening the suture thread 25, the suture thread 25 does not go slack even when the suture tool 16 is placed inside the stomach 43.

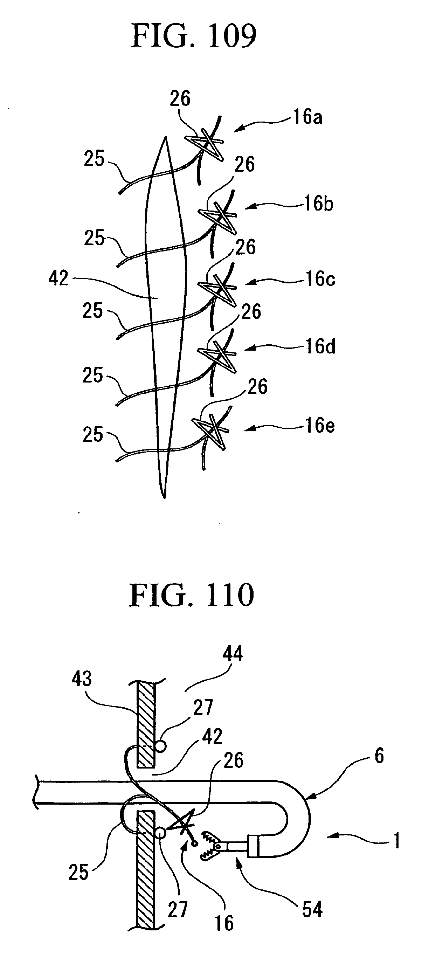

By tightening the two suture tools 16 in order, the perforation 42 is sutured as shown in FIG. 108. In the case of suturing the perforation 42 with three or more suture tools 16 such as when making a large incision in the stomach 43, the suture tools 16 may be tightened in order from one end of a row thereof. For example, in the example shown in FIG. 109, the suture tools 16 are tightened in the order of suture tool 16a, suture tool 16b, suture tool 16c, suture tool 16d, and suture tool 16e. Since the size of the perforation 42 can gradually be made smaller by suturing the perforation 42 from one end, the suturing becomes easy. Also, it is acceptable to initially tighten the suture tool 16 that is in the middle of the row of suture tools 16, and next tighten the suture tools 16 that are positioned between the middle suture tool 16 that has been tightened and the suture tools 16 at the ends. In the example of FIG. 109, the suture tool 16c is initially tightened, and next the suture tool 16b and the suture tool 16d are tightened, and finally the suture tool 16a and the suture tool 16e are tightened. By always suturing the middle portion of the opening, shifting of the suturing position is minimized.

According to this example, by mounting the suture tool 16 in a manner straddling the intended incision line 300 before making an incision in the stomach 43, it is possible to prevent shifting of the suture position. In conventional methods, after making the incision, the needle is thrust in while confirming the suture position by sight. As a result, the suture position readily shifts, and careful attention has been required in order to prevent leaks from the perforation. However, since shifting of the suture position is prevented in the present example, the procedure is easy. Also, since endoscope procedures are restricted to approaches from one direction, the field of vision has been limited, and the suture position has had to be selected in consideration of the tissue clearance and extension. But in the present example, it is possible to reliably close the perforation by specifying the puncture positions 301, 302, 303, 304 before making an incision.

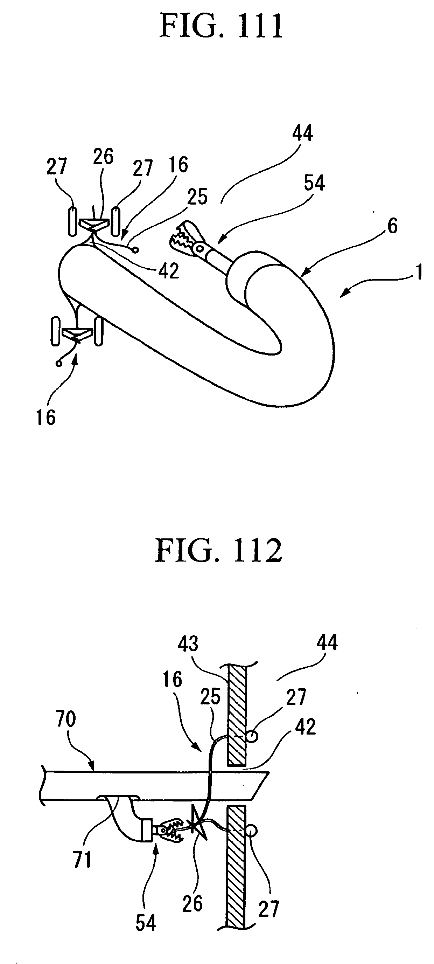

Here, there are times when the endoscope insertion part 6 is fed from the perforation 42 to the abdominal cavity 53, and by excising a large tissue is brought out from the perforation 42 to outside of the body through the stomach 43. When the perforation 42 is formed in accordance with the size of the tissue, the size of the perforation 42 can at times be greater than the diameter of the endoscope insertion part 6. In this case, when performing treatment in the abdominal cavity 53, by supplying a gas from the distal end portion of the endoscope insertion part 6 to swell the abdominal cavity, gas enters the stomach 43 from the endoscope insertion part 6 and the gap of the perforation 42. In order to prevent the gas from flowing into the stomach 43, the suture tool 16 may be lightly tightened to narrow the size of the perforation 42 narrowed to the extent of the size of the endoscope insertion part 6. As shown in FIG 110, when the endoscope insertion part 6 is fed from the perforation 42 to the abdominal cavity 53, the stopper 26 is drawn out to the abdominal cavity 53 by the forceps 54 that are passed through the endoscope insertion part 6. As shown in FIG 111, when the stopper 26 is pressed against the outer wall of the stomach 43 and the suture tool 16 is lightly tightened, the gap of the perforation 42 becomes smaller. Thereafter, when gas is supplied from the distal end portion of the endoscope insertion part 6, the abdominal cavity 44 swells. When the procedure is completed, the endoscope insertion part 6 is drawn into the stomach 43. The stopper 26 is then pulled into the stomach 43 with the forceps 54, and the suture tool 16 is tightened, whereby the perforation 42 is sutured.

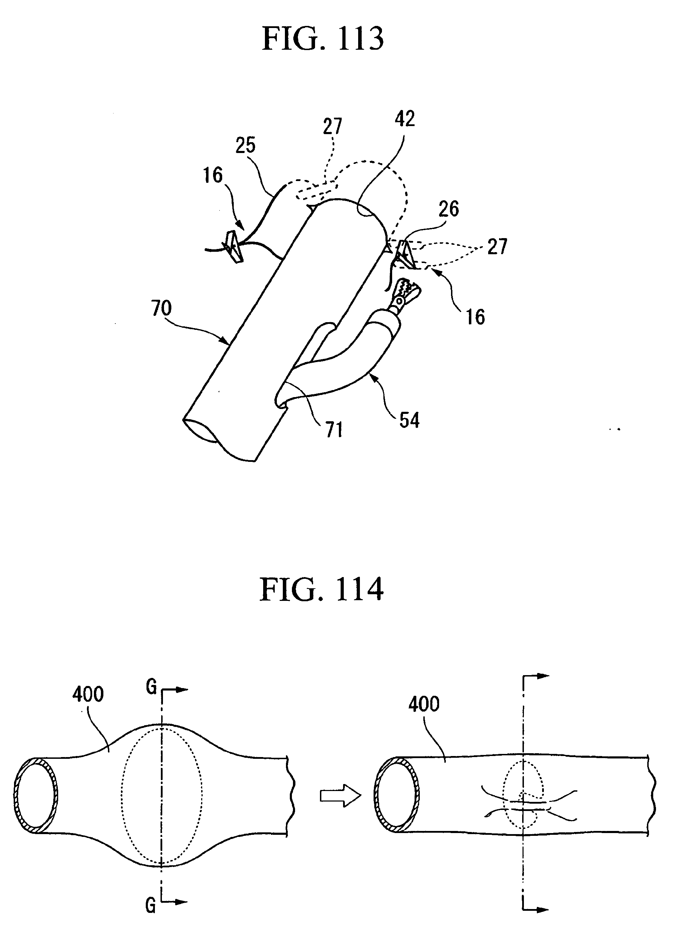

Also, as shown in FIG 112, in the case of performing a procedure by passing the endoscope insertion part 6 in an overtube 70, the distal end of the overtube 70 is projected from the perforation 42 into the abdominal cavity 53. As shown in FIG 113, the forceps 54 are fed from the side hole 70A of the overtube 70 on the interior side of the stomach 43, and the suture tool 16 is lightly tightened to narrow the perforation 42 to the extent of the outer diameter of the overtube 70. The endoscope insertion part 6 is projected from the distal end of the overtube 70, and the abdominal cavity 53 is swelled with gas to perform the treatment. When the procedure is completed, the overtube 70 is drawn back from the perforation 42. Thereafter, when the suture tool 16 is tightened by the forceps 54, the perforation 42 is sutured.

Note that the present invention can be widely applied without being limited to the aforesaid embodiments.

For example, the endoscope 1 may be inserted from the anus into the large intestine that is one example of a hollow organ. In this case, the perforation in the large intestine and the like is sutured.

In the first and second examples, the needle 14 is inserted approximately perpendicular with respect to the muscle layer 47, but it may also be inserted at a predetermined angle. Thereby, the suture thread 25 passes through the muscle layer 47 obliquely with respect to the axial line of the perforation 42. In this case, the same effect is obtained.

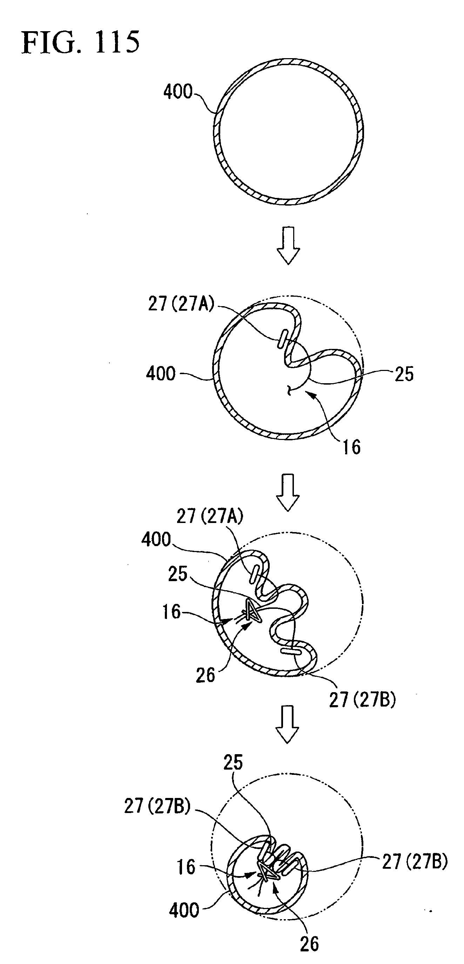

Also, various application examples are conceivable such as suturing the diameter of a hollow organ 400 as shown in FIG 114, without being limited to perforation sutures and membrane sutures. In this case, as shown in FIG. 115, anchors 27A, 27B are placed at an interval in the inner circumferential direction of the hollow organ 400, and by tightening them, the hollow organ wall is sutured so as to be folded over. By forming a plurality of such sutures along the axis direction of the hollow organ, it is possible to reduce the diameter of a hollow organ over a desired length.

In the case of the endoscope insertion part 6 having two channels 9, a suture instrument 11 may be passed through each channel 9. In this case, the anchor 27 of the suture tool 16 is housed in the respective needle 14 of each suture instrument 11.

The number of anchors may be a plurality and is not limited to two.

### [INDUSTRIAL APPLICABILITY]

The suture method and suture instrument according to this invention may be preferably utilized for medical applications.

## Claims

1. A suture instrument (201) that is inserted into a body and ejects an elongated anchor (206A) to be engaged to tissue, comprising:
an elongated member (13) that extends from a proximal end on a hand side to a remote end that is introduced to tissue and has flexibility;
a needle (202) that is provided at the remote end of the elongated member (13), has at the distal end a sharp tip that is capable of puncturing tissue, is capable of housing a plurality of the anchors (206A, 206B) inside, and is provided at the sharp tip with an opening portion (203) that is capable of pushing out the anchors (206A, 206B);
a pusher (20) that pushes out the anchors (206A, 206B) from the needle (202) through the opening portion (203); and
a push-out control portion (209) that is capable of engaging and disengaging with at least one of the anchors (206A, 206B) to cause the anchors (206A, 206B) to be pushed out one at a time from the opening portion (203), and is provided in the needle (202) or the pusher (20);
**characterized in that**:
the push-out control portion (209) is mounted on the needle (202) and has a projection (209A) that passes through the needle (202) to protrude toward the inner side thereof, and
a recessed portion (207) that is capable of engaging and disengaging with the projection (209A) is provided on the anchors (206A, 206B).

2. The suture instrument (201) according to claims 1, wherein
two of the anchors (206A, 206B) are housed in the needle (202), and the recessed portion (207) is formed on only one of the anchors (206A, 206B).

## Patentansprüche

1. Nähgerät (201), das in einen Körper eingeführt ist und einen langgestreckten Anker (206A) auswirft, der dazu vorgesehen ist, mit Gewebe zusammenzuwirken, wobei das Nähgerät umfasst:
ein langgestrecktes Element (13), das sich von einem proximalen Ende an einer Handseite zu einem entfernten Ende erstreckt, das in Gewebe eingeführt ist und Flexibilität hat;
eine Nadel (202), die an dem entfernten Ende des langgestreckten Elements (13) angebracht ist, an dem distalen Ende eine scharfe Spitze hat, die dazu in der Lage ist, Gewebe zu durchdringen, dazu in der Lage ist, eine Mehrzahl der Anker (206A, 206B) in ihrem Inneren aufzunehmen und an der scharfen Spitze mit einem Öffnungsabschnitt (203) versehen ist, der dazu in der Lage ist, die Anker (206A, 206B) herauszudrücken;
einen Drücker (20), der die Anker (206A, 206B) durch den Öffnungsabschnitt (203) aus der Nadel (202) herausdrückt; und
einen Herausdrück-Steuerungsabschnitt (209), der dazu in der Lage ist, mit wenigstens einem der Anker (206A, 206B) verbunden oder von wenigstens einem der Anker (206A, 206B) getrennt zu werden, um zu veranlassen, dass die Anker (206A, 206B) einer nach dem anderen aus dem Öffnungsabschnitt (203) herausgedrückt werden und der in der Nadel (202) oder dem Drücker (20) vorgesehen ist;
**dadurch gekennzeichnet, dass**
der Herausdrück-Steuerungsabschnitt (209) an der Nadel (202) angebracht ist und einen Vorsprung (209A) hat, der durch die Nadel (202) hindurchführt, um in Richtung der Innenseite davon zu ragen, und
ein ausgesparter Abschnitt (207), der dazu in der Lage ist, mit dem Vorsprung (209A) verbunden oder von dem Vorsprung (209A) getrennt zu werden, an den Ankern (206A, 206B) vorgesehen ist.

2. Nähgerät (201) gemäß Anspruch 1, wobei
zwei der Anker (206A, 206B) in der Nadel (202) untergebracht sind und der ausgesparte Abschnitt (207) nur an einem der Anker (206A, 206B) ausgebildet ist.

## Revendications

1. Instrument (201) de suture qui est inséré dans un corps et éjecte un ancrage (206A) allongé destiné à être engagé avec un tissu, comprenant :
un élément (13) allongé qui s'étend d'une extrémité proximale sur un côté de main jusqu'à une extrémité distante qui est introduite dans un tissu et a une flexibilité ;
une aiguille (202) qui est prévue au niveau de l'extrémité distante de l'élément (13) allongé, a, à l'extrémité distale, une pointe effilée qui est apte à percer un tissu, est apte à recevoir une pluralité des ancrages (206A, 206B) à l'intérieur, et est prévue au niveau de la pointe effilée avec une partie (203) d'ouverture qui est apte à faire sortir par poussée les ancrages (206A, 206B) ;
un poussoir (20) qui fait sortir par poussée les ancrages (206A, 206B) de l'aiguille (202) à travers la partie (203) d'ouverture ; et
une partie (209) de contrôle de sortie par poussée qui est apte à s'engager avec et se désengager d'au moins un des ancrages (206A, 206B) pour faire en sorte que les ancrages (206A, 206B) soient sortis par poussée un à la fois à partir de la partie (203) d'ouverture, et est prévue dans l'aiguille (202) ou le poussoir (20) ;
**caractérisé en ce que** :
la partie (209) de contrôle de sortie par poussée est montée sur l'aiguille (202) et a une projection (209A) qui passe à travers l'aiguille (202) pour faire saillie vers le côté intérieur de celle-ci, et
une partie (207) d'évidement qui est apte à s'engager avec et se désengager de la projection (209A) est prévue sur les ancrages (206A, 206B).

2. Instrument (201) de suture selon la revendication 1, dans lequel deux des ancrages (206A, 206B) sont reçus dans l'aiguille (202), et la partie (207) d'évidement est formée sur un seul des ancrages (206A, 206B).
